(19) Europäisches Patentamt
European Patent Office
Office européen des brevets

(11) Numéro de publication: **0 241 363 B1**

(12) **FASCICULE DE BREVET EUROPEEN**

(45) Date de publication de fascicule du brevet: **25.09.91**

(51) Int. Cl.[5]: **C07H 21/00**, C07H 19/173, C07H 19/073, C07H 19/12

(21) Numéro de dépôt: **87400739.6**

(22) Date de dépôt: **03.04.87**

Demande divisionnaire 91200556.8 déposée le 03/04/87.

Le dossier contient des informations techniques présentées postérieurement au dépôt de la demande et ne figurant pas dans le présent fascicule.

(54) **Dérivés de nucléosides et leur utilisation pour la synthèse d'oligonucléotides.**

(30) Priorité: **08.04.86 FR 8604990**

(43) Date de publication de la demande: **14.10.87 Bulletin 87/42**

(45) Mention de la délivrance du brevet: **25.09.91 Bulletin 91/39**

(84) Etats contractants désignés: **AT BE CH DE ES FR GB GR IT LI LU NL SE**

(56) Documents cités:
**EP-A- 0 090 789**

**Tetrahedron (1981), vol. 37, pp 363-369**

**Nucleic Acid Research, vol 15 (2), 1987, 397 ff**

(73) Titulaire: **COMMISSARIAT A L'ENERGIE ATOMIQUE Etablissement de Caractère Scientifique Technique et Industriel**
**31/33, rue de la Fédération**
**F-75015 Paris(FR)**

(72) Inventeur: **Molko, Didier**
**11, Avenue de la Gare**
**F-38120 Tullins(FR)**
Inventeur: **Schulhof, Jean-Claude**
**49 Le Hameau Fleuri**
**Montbonnot F-38330 Saint Ismier(FR)**
Inventeur: **Teoule, Robert**
**52, rue Thiers**
**F-38000 Grenoble(FR)**

(74) Mandataire: **Mongrédien, André et al**
**c/o BREVATOME 25, rue de Ponthieu**
**F-75008 Paris(FR)**

**EP 0 241 363 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouveaux dérivés de nucléosides et leur utilisation pour la synthèse d'oligonucléotides.

De façon plus précise, elle concerne des dérivés de nucléosides formés à partir de bases pyrimidiques ou puriques comportant des groupements $NH_2$ exocycliques, c'est-à-dire des nucléosides formés à partir d'adénine, de guanine ou de cytosine, qui sont utilisables en particulier pour la synthèse d'oligonucléotides.

La synthèse d'oligonucléotides consiste à lier entre eux des nucléosides par un groupement phosphate pour former une chaîne d'ADN (acide désoxyribonucléique) ou d'ARN (acide ribonucléique). Dans cet assemblage, les groupements phosphates internucléotidiques relient toujours la fonction hydroxyle en position 3' d'un nucléoside à la fonction hydroxyle en position 5' d'un autre nucléoside. Ainsi, lors de la réaction de synthèse, seules les extrémités 3' et 5' des nucléosides sont sollicitées et la base nucléique (purique ou pyrimidique) utilisée ne doit pas intervenir au cours de cet assemblage.

Lorsque ces bases comprennent des groupements $NH_2$ exocycliques, il est nécessaire de protéger ces groupements lors de la synthèse des oligonucléotides car ceux-ci sont trop réactifs et risquent d'interférer avec les réactions de synthèse.

Cette protection des groupements $NH_2$ exocycliques doit répondre à certaines caractéristiques :

- elle doit être sélective et facile à réaliser,
- elle ne doit pas induire de modifications de réactivité des autres sites du nucléoside, et être stable pendant toutes les étapes de la synthèse oligonucléotidique, et
- elle doit pouvoir être éliminée ensuite dans des conditions douces sans détruire l'oligonucléotide qui vient d'être synthétisé.

Les groupements $NH_2$ exocycliques des nucléosides ont le plus souvent été protégés sous la forme d'amides, par exemple au moyen de groupes benzoyle ou anisoyle dans le cas de l'adénine et de la cytosine comme il est décrit par M. SCHALLER et al dans J. Amer. Chem. Soc. (1963), vol. 85, p. 3821-3827, et au moyen du groupe isobutyryle dans le cas de la guanine comme il est décrit par H.BÜCHI et M. KHORANA dans J. Mol. Biol. (1972), vol. 72, p. 251-288.

On a aussi envisagé de protéger les groupements $NH_2$ exocycliques des nucléosides par d'autres groupes, par exemple par des groupes alkylcarbonyle comme il est décrit par Seliger et al dans Chem. Ber., 111, p. 3732-3739 (1978) dans le cas de la cytosine, ou par le groupe propionyle dans le cas de la guanine, comme il est décrit par Kamimura et al dans Tetrahedron Letters, vol. 24, n° 27, p. 2775-2778 (1983).

Koster et al dans Tetrahedron (1981), vol. 37, p. 363 à 369 étudie aussi la possibilité d'utiliser comme groupe protecteur des groupes aminoexocycliques de désoxynucléosides des groupes N-acyle plus stables tels que des groupes aromatiques.

Ces groupes protecteurs peuvent être éliminés en fin de synthèse par action de l'ammoniaque à 28%, pendant 17 h, à une température de 60°C, comme il est préconisé. Cependant la RMN du proton montre que dans ces conditions on n'élimine pas tous les groupements isobutyryle de la guanine ; il est donc préférable de porter le temps de réaction à 72 heures, toujours à une température de 60°C.

Ce mode d'élimination des groupes protecteurs constitue un inconvénient car les conditions mises en oeuvre ne sont pas suffisamment douces pour pouvoir être utilisées avec des nucléosides modifiés, peu stables en milieu alcalin, comme c'est le cas par exemple de la dihydro-5,6 thymidine.

Aussi, des recherches ont été entreprises sur la possibilité d'utiliser d'autres groupements acyle plus faciles à éliminer, utilisables en particulier pour la synthèse d'oligonucléotides à partir de nucléosides labiles par la méthodologie de synthèse sur support qui consiste à fixer le premier nucléoside de la chaîne sur un support, généralement en silice, et à effectuer ensuite successivement des cycles de condensation pour fixer sur le premier nucléoside les autres nucléosides dans l'ordre voulu. L'utilisation de groupements acyle plus faciles à éliminer permet par ailleurs d'obtenir un meilleur rendement de déprotection. Ce point est très important car la présence de bases incomplètement déprotégées présente un inconvénient pour l'utilisation des produits obtenus.

La présente invention a précisément pour objet de nouveaux dérivés de nucléosides comportant des groupements protecteurs de type acyle, susceptibles d'être éliminés facilement.

Les dérivés de nucléosides de l'invention répondent à la formule :

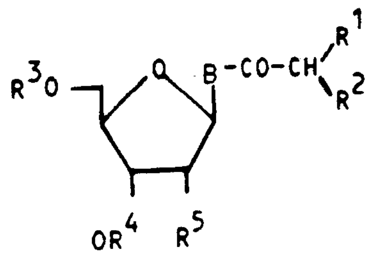

(I)

dans laquelle B représente un radical bivalent choisi parmi :

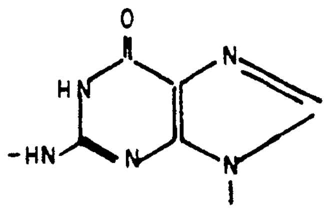

(II) ;

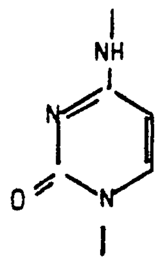

(III) ou

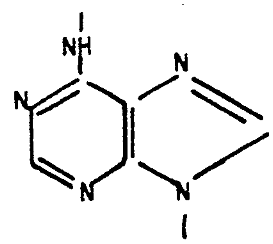

(IV)

relié au groupement CO par son groupement NH- exocyclique;

- $R^1$ représente un atome d'hydrogène ou un radical méthyle ou éthyle ;
- $R^2$ représente un radical choisi dans le groupe constitué des radicaux méthoxy et phénoxy non substitué ou substitué par un ou des groupements choisis parmi $NO_2$, CN, méthoxy, éthoxy, phénoxy

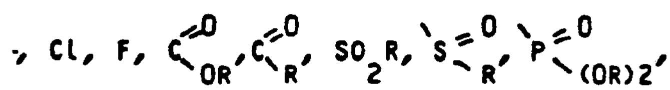

méthyle, éthyle ou phényle substitué ou non, SR avec R représentant un radical méthyle, éthyle ou phényle

- $R^3$ représente un atome d'hydrogène, un radical labile en milieu acide ou le radical de formule :

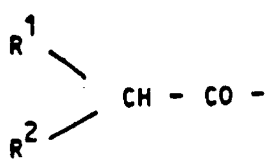

dans laquelle $R^1$ et $R^2$ ont la signification donnée ci-dessus,

- $R^4$ représente un atome d'hydrogène, un radical phosphoré ou le radical :

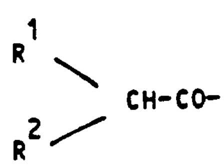

dans lequel $R^1$ et $R^2$ ont la signification donnée ci-dessus ; et

- $R^5$ représente un atome d'hydrogène ou le radical OH protégé ou non.

A titre d'exemple, les radicaux labiles en milieu acide susceptibles d'être utilisés pour former $R^3$ dans le composé de formule (I) sont en particulier des radicaux utilisables en synthèse oligonucléotidique tels que :

- les radicaux trityle répondant à la formule :

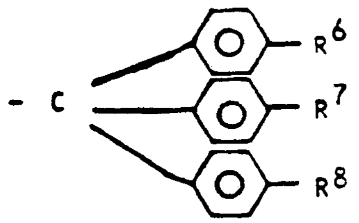

(V)

dans laquelle $R^6$ , $R^7$ et $R^8$ qui peuvent être identiques ou différents, représentent un atome d'hydrogène, un radical alkyle ou un radical alkoxy, par exemple le radical monométhoxytrityle ou le radical trityle de formule (V) dans laquelle $R^6$ et $R^7$ représentent le radical méthoxy et $R^8$ représente un atome d'hydrogène ;

- le radical pixyle ou phényl-9 xanthènyle.

A titre d'exemple, les radicaux phosphorés susceptibles d'être utilisés pour former $R^4$ dans le composé de formule (I) sont également des radicaux utilisables en synthèse oligonucléotidique tels que le radical de formule :

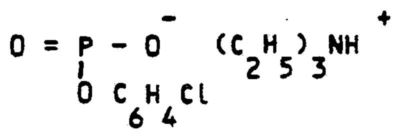

(VI),

le radical de formule :

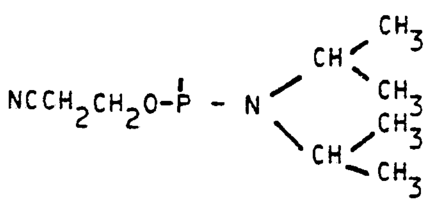

(VII)

le radical phosphonate de formule :

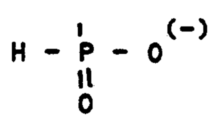

Selon l'invention, lorsque $R^5$ représente le radical OH protégé, on utilise comme groupe protecteur de OH les groupes habituellement utilisés dans la synthèse des ribonucléotides.

Les dérivés de nucléosides de l'invention sont ainsi les produits de l'union 1°) d'une base formée par la guanine, la cytosine ou l'adénine et 2°) du ribose ou du désoxyribose, les nucléosides étant modifiés au moins sur le groupement $NH_2$ exocyclique de leur base par un groupement

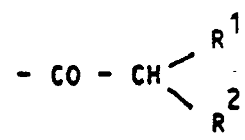

Ils peuvent également être modifiés par ce même groupement sur les positions 3' et 5' du désoxyribose ou 2', 3' et 5' du ribose, ou les positions 3' et 5' du ribose ou du désoxyribose peuvent être modifiées par d'autres groupements qui sont les groupements labiles $R^3$ pour la position 5' et le groupement phosphoré $R^4$ sur la position 3' du ribose ou du désoxyribose.

Les groupements acyle de formule

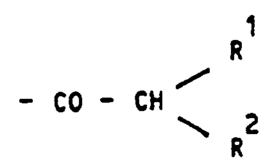

utilisés dans l'invention sont particulièrement intéressants pour la synthèse des nucléotides car ils peuvent être éliminés facilement en fin d'opération, par exemple par traitement à l'ammoniaque pendant 2 à 8 h selon le groupement utilisé, à la température ambiante, ce qui permet de libérer simultanément le polynucléotide du support sur lequel il a été synthétisé, lorsqu'on utilise la méthode de synthèse sur support.

Dans le groupe protecteur de formule

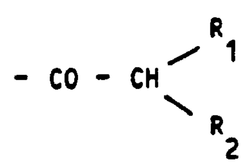

utilisé dans l'invention $R^1$ peut présenter un atome d'hydrogène ou un radical méthyle ou ethyle et $R^2$ peut représenter un radical méthoxy ou un phénoxy éventuellement substitué par différents groupements.

Selon l'invention, on choisit le groupement protecteur des groupements $NH_2$ exocycliques en fonction de la base utilisée afin d'obtenir la résistance au traitement alcalin souhaité.

Généralement, lorsque la base utilisée est la guanine, $R^1$ représente un atome d'hydrogène et $R^2$ est un radical methoxy ou un radical phenoxy éventuellement substitué.

A titre d'exemple, le radical $R^2$ peut être le radical phénoxy, le radical méthoxy ou le radical chloro-2 phénoxy.

Lorsque la base utilisée est l'adénine, $R^1$ représente de préférence un atome d'hydrogène et $R^2$ représente un radical phenoxy éventuellement substitué, par exemple le radical phénoxy.

L'utilisation des groupes protecteurs mentionnés cidessus permet d'obtenir une réduction du temps de déprotection des oligonucléotides obtenus par assemblage des nucléosides de l'invention, puisque ce temps peut être seulement de 2 à 8 h selon le groupement utilisé, au lieu des 17 à 72 h nécessaires antérieurement. On peut par ailleurs opérer dans des conditions réactionnelles plus douces puisqu'on réalise la déprotection à température ambiante alors qu'il était nécessaire auparavant de chauffer à 60° C. De plus, l'utilisation de ces groupes protecteurs éliminables plus facilement permet d'incorporer lors de la synthèse des oligonucléotides des bases nucléiques modifiées sensibles à des conditions alcalines plus violentes, par exemple de synthétiser des fragments d'ADN porteurs de ligands sensibles à des anticorps.

Bien que l'invention s'applique aux nucléosides dérivés du ribose et aux nucléosides dérivés du désoxyribose, on l'utilise de préférence pour les nucléosides dérivés du désoxyribose, c'est-à-dire aux dérivés de formule (I) dans laquelle $R^5$ est un atome d'hydrogène.

Les dérivés de nucléosides de l'invention peuvent être préparés par des procédés classiques, analogues à ceux utilisés pour fixer les groupements benzoyle et anisoyle sur les nucléosides à base d'adénine ou de cytosine. Dans ces procédés, on part du nucléoside de la guanine, de la cytosine ou de

l'adénine, que l'on fait réagir avec le chlorure d'acide de formule :

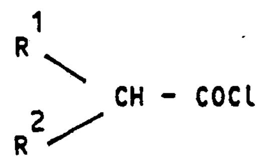

ou l'anhydride d'acide de formule :

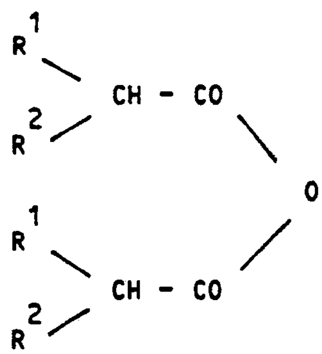

Lors de cette réaction, l'anhydride ou le chlorure d'acide réagit également avec les groupements hydroxyle en position 3′ et 5′ du ribose ou du désoxyribose et l'on obtient ainsi le dérivé du nucléoside triprotégé soit le dérivé de formule (I) dans lequel $R^3$ et $R^4$ représentent tous deux le radical :

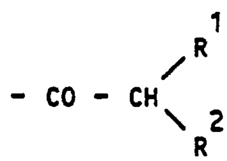

On peut toutefois éliminer ces radicaux en position 3′ et 5′ par hydrolyse sélective, ce qui permet d'obtenir les dérivés de nucléosides de formule (I) dans laquelle $R^3$ et $R^4$représentent des atomes d'hydrogène.

On peut préparer les dérivés de nucléosides de formule (I) dans laquelle $R^3$ représente un radical trityle de formule (V), par exemple le groupement diméthoxytrityle, et $R^4$ représente un atome d'hydrogène en faisant réagir les dérivés des nucléosides obtenus précédemment avec le chlorure de trityle correspondant dans un solvant approprié.

Les dérivés de nucléosides répondant à la formule (I) dans laquelle $R^3$ représente un groupement trityle et $R^4$ représente soit le radical de formule (VI), soit le radical de formule (VII), soit un radical de formule :

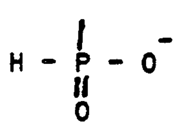 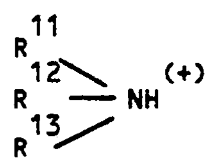

dans laquelle $R^{11}$, $R^{12}$ et $R^{13}$ qui peuvent être identiques ou différents, sont des radicaux alkyle, par exemple éthyle, peuvent être préparés par des procédés classiques à partir des dérivés de nucléosides de formule (I) dans laquelle $R^3$ représente un radical trityle et $R^4$ représente un atome d'hydrogène.

Dans le cas où $R^4$ représente, par exemple, le radical de formule (VI), on fait réagir ce dérivé de nucléoside avec du bistriazolidate de chloro-4 phénylphosphoryle dans un solvant approprié. Le bistriazolidate de chloro-4 phénylphosphoryle peut être préparé par addition de dichlorophosphate de chloro-4 phényle à une suspension de triazole et de triéthylamine dans du dioxanne.

Lorsque $R^4$ représente, par exemple, le radical de formule (VII), on peut faire réagir le dérivé de nucléoside avec du $\beta$ -cyanoéthyl-monochloro N,N-diisopropyl amino phosphoramidite dans un solvant approprié en présence de diisopropyléthylamine.

Lorsque $R^4$ représente, par exemple, le radical de formule :

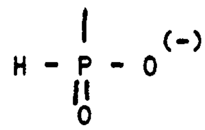
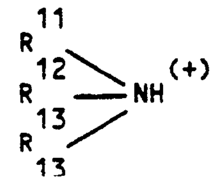

dans laquelle $R^{11}$, $R^{12}$ et $R^{13}$ qui peuvent être identiques ou différents, sont des radicaux alkyle, on peut faire réagir le dérivé de nucléoside avec de la chloro-2 benzo(5,6-a) [dioxo-1,3 phosphor-2 inone-4] puis avec un sel de trialkyl ammonium tel que l'acétate de triéthyl ammonium.

Les dérivés de nucléosides obtenus par ces trois méthodes peuvent être utilisés pour la synthèse d'oligonucléotides soit par synthèse phosphotriester dans le cas où $R^4$ est le radical de formule (VI), soit par synthèse phosphoramidite dans le cas où $R^4$ est le radical de formule (VII), soit par synthèse H-phosphonate dans le cas où $R^4$ est le radical de formule :

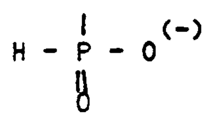
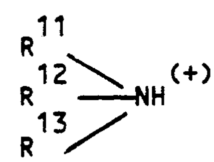

en utilisant également pour l'assemblage des chaîne oligonucléotidiques d'autres nucléosides, par exemple ceux correspondant à la thymidine et à la désoxy-2' uridine, ou encore des nucléosides comportant des bases labiles en milieu alcalin ou d'autres nucléosides labiles en milieu alcalin.

Le procédé, selon l'invention, de synthèse d'oligonucléotides, comprend :

1) au moins un cycle de condensation dans lequel on condense sur un dérivé de nucléoside ou sur un oligonucléotide un dérivé de nucléoside de formule :

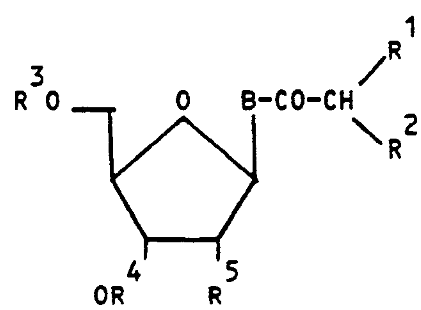
(I)

dans laquelle B représente un radical bivalent choisi parmi :

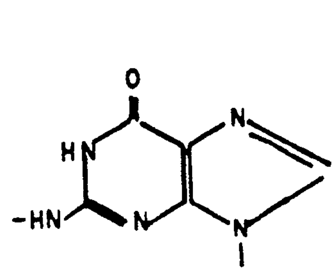 (II) ; 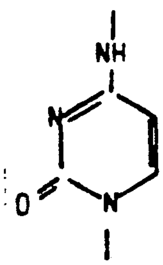 (III) ou 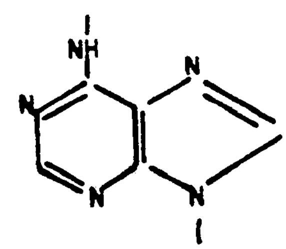 (IV)

relié au groupement CO par son groupement NH- exocyclique ;

- $R^1$ représente un atome d'hydrogène ou un radical méthyle ou éthyle;
- $R^2$ représente un radical choisi dans le groupe constitué des radicaux méthoxy et phénoxy non substitué ou substitué par un ou des groupements choisis parmi $NO_2$, CN, méthoxy, éthoxy, phénoxy

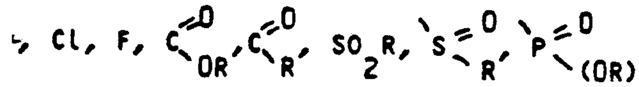

méthyl, éthyle ou phényle substitué ou non, SR avec R représentant un radical méthyle, éthyle ou phényle ;
- $R^3$ représente un radical labile en milieu acide,
- $R^4$ représente un radical phosphoré, et
- $R^5$ représente un atome d'hydrogène ; et

2°) une étape d'élimination du ou des groupes protecteurs de formule :

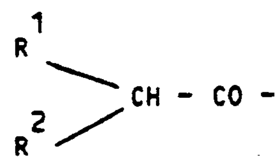

dans Laquelle $R^1$ et $R^2$ sont tels que définis ci-dessus, par exemple par mise en contact de l'oligonucléotide avec de l'ammoniaque à la température ambiante.

La synthèse oligonucléotidique peut être effectuée soit par des méthodes en solution, soit par des méthodes de synthèse sur support. On utilise de préférence les méthodes de synthèse sur support car celles-ci sont mieux adaptées à l'utilisation de nucléosides plus labiles, sans qu'il y ait une perte de rendement au cours de l'assemblage.

Les nucléosides de l'invention peuvent donc trouver des applications intéressantes en tant que produits de base destinés à la synthèse de fragments d'ADN ou d'ARN. Ils peuvent également convenir pour incorporer dans des oligonucléotides de synthèse des bases modifiées fragiles, ce qui peut concerner en particulier les produits de radiolyse gamma de l'ADN ainsi que les produits de photolyse.

Les nucléosides de l'invention peuvent permettre également l'accès à de nouvelles molécules à activité antivirale et à de nouvelles sondes d'ADN.

Les exemples suivants de préparation et d'utilisation des nucléosides conformes à l'invention sont donnés bien entendu à titre non limitatif pour illustrer l'invention. Les Composés 5,6,11,14 et 19 ne font pas partie de l'invention.

EXAMPLE 1 : Préparation de ($N_2$ -phénoxyacétyl)-désoxy-2′ guanosine (composé n°)1

On sèche 1080 mg (4 mmol) de désoxy-2′ guanosine par additions et évaporations successives de pyridine anhydre, puis on les met en suspension dans 20 ml de pyridine anhydre et on introduit la suspension dans un ballon. On refroidit le ballon au bain de glace et on y ajoute lentement 6 équivalents (4,25 g ; 24 mmol) de chlorure de phénoxyacétyle à 0°C. On laisse ensuite la réaction se poursuivre à la température ambiante pendant 90 min. Un précipité blanc de chlorure de pyridinium apparaît dans le milieu réactionnel tandis que celui-ci prend une coloration orangée à brune. On obtient ainsi le dérivé triprotégé du nucléoside de départ, c'est-à-dire le dérivé de formule (I) dans laquelle B représente le radical de formule (II), dérivé de la guanine, $R^1$ représente un atome d'hydrogène, $R^2$ représente le radical phénoxy, $R^3$ et $R^4$ représentent le radical phénoxyacétyle et $R^5$ est un atome d'hydrogène.

On détruit alors l'excès de chlorure d'acide à 0°C par 2 ml d'eau bidistillée, ce qui solubilise le milieu réactionnel puis on le dilue par 100 ml de chloroforme. On lave 4 fois la phase chloroformique par 50 ml de solution aqueuse à 5% de bicarbonate de sodium pour éliminer l'acide phénoxyacétique formé. On sèche alors la phase chloroformique sur du sulfate de sodium, on évapore le solvant et on obtient ainsi un résidu orange. On dissout celui-ci dans 100 ml de pyridine refroidis à 0°C, puis on ajoute 100 ml de soude 0,2 N à 0°C. On effectue ainsi l'hydrolyse sélective des positions 3′ et 5′ en 20 min. On neutralise alors le milieu

au moyen de la résine échangeuse de cations, Dowex 50W-X8 d'une granulométrie de 100 à 200 mesh (0,074 à 0,149 mm), sous forme pyridinium. Après filtration et rinçage de la résine, on évapore à sec le filtrat.

On isole ensuite la $N_2$-phénoxyacétyl-désoxy-2′ guanosine formée par chromatographie sur une colonne de silice (3 cm de diamètre, 15 cm de hauteur) que l'on élue par un gradient de chloroforme-méthanol. L'évaporation des fractions contenant le produit recherché permet de recueillir 250 mg de $N_2$-phénoxyacétyl-désoxy-2′ guanosine, ce qui correspond à un rendement de 15%.

On contrôle l'identité et la pureté du produit obtenu par résonance magnétique nucléaire à 250 MHz, par chromatographie en couche mince et par spectrométrie de masse. On obtient les résultats suivants :

- $R_F$ = 0,36 avec le mélange de migration chloroforme-méthanol (80/20 en vol),
- (M + H) pic moléculaire (m/e : 402 - 13%) ; guanine phénoxyacétylée (m/e : 286 - 51%).

EXEMPLE 2 : Préparation de [-$N_2$-(chloro-2 phénoxy)acétyl]-désoxy-2′ guanosine (composé n°)2

On sèche 1080 mg (4 mmol) de désoxy-2′ guanosine comme dans l'exemple 1, puis on les met en suspension dans 20 ml de pyridine anhydre et on les introduit dans un ballon placé dans un bain d'eau glacée. On ajoute lentement à 0° C, 6 équivalents (5,1 g ; 24 mmol) de chlorure de (chloro-2-phénoxy)-acétyle. On laisse la réaction se poursuivre à la température ambiante pendant 150 min. Une coloration verte à marron apparaît dans le milieu réactionnel et on forme ainsi le dérivé triprotégé du nucléoside de départ, soit le dérivé de formule (I) dans laquelle B représente le radical de formule (II) dérivé de la guanine, $R^1$ représente un atome d'hydrogène, $R^2$ représente le radical chloro-2 phénoxy, $R^3$ et $R^4$ représentent le radical chloro-2 phénoxyacétyle et $R^5$ est un atome d'hydrogène.

On détruit l'excès de chlorure d'acide à 0° C par 2 ml d'eau distillée qui solubilisent le milieu réactionnel. On le dilue alors par 100 ml de chloroforme et on lave cette phase chloroformique par 4 fois 50 ml d'une solution aqueuse à 5% de bicarbonate de sodium pour éliminer l'acide chlorophénoxyacétique. On sèche la phase chloroformique sur du sulfate de sodium, on évapore le solvant et l'on obtient ainsi un résidu orangé. On dissout ce résidu dans 100 ml de pyridine, on place la solution obtenue dans un bain d'eau glacée et on lui ajoute 100 ml de soude 0,2 N, ce qui donne un mélange titrant 0,1N et permet d'hydrolyser sélectivement les positions 3′ et 5′ du nucléoside en 20 min. On neutralise alors le milieu par la résine échangeuse de cations Dowex 50W-X8 utilisée dans l'exemple 1 sous forme pyridinium. On filtre et on rince la résine, puis on évapore à sec le filtrat. On obtient ainsi la [$N_2$-(chloro-2 phénoxy)acétyl]-désoxy-2′ guanosine qui est soluble uniquement dans la pyridine.

On la purifie par chromatographie sur colonne de silice en utilisant un gradient chloroforme-méthanol. On isole ainsi 220 mg du composé n°2, ce qui correspond à un rendement de 13%. On caractérise le composé par chromatographie en couche mince et par spectrométrie de masse.

Les résultats obtenus sont les suivants :

- $R_F$ = 0,4 dans un mélange de migration chloroforme-méthanol (80/20 en volume),
- (M + H) : pic moléculaire (m/e : 436 - 17%) ; guanine chloro-2 phénoxyacétylée (m/e : 320 - 44%).

La pureté du produit est confirmée par analyse par résonance magnétique nucléaire à 250 MHz.

EXEMPLE 3 : Préparation de( $N_2$-méthoxyacétyl)-désoxy-2′guanosine (composé n°3)

On sèche 5,4 g (20 mmol) de désoxyguanosine puis on les met en suspension dans 100 ml de pyridine anhydre. On refroidit à 0° C et on ajoute lentement 4,5 équivalents (10 g ; 90 mmol) de chlorure de méthoxyacétyle. On laisse la réaction se poursuivre à la température ambiante pendant 3 h pour former le dérivé triprotégé du nucléoside de départ, soit le dérivé de formule (I) dans laquelle B représente le radical de formule (II) dérivé de la guanine, $R^1$ représente un atome d'hydrogène, $R^2$ représente le radical méthoxy, $R^3$ et $R^4$ représentent le radical méthoxyacétyle et $R^5$ représente un atome d'hydrogène.

On détruit l'excès de chlorure d'acide par du méthanol pendant 30 min, ce qui produit du méthoxyacétate de méthyle de basse température d'ébullition (129-130° C). On évapore les solvants et on reprend le résidu par du chloroforme, puis on le lave par une phase aqueuse à 5% de bicarbonate de sodium. On sèche la phase chloroformique sur du sulfate de sodium, puis on l'évapore, ce qui conduit à l'obtention d'un résidu orangé qui correspond au dérivé triprotégé.

On purifie le dérivé par chromatographie sur colonne de silice (diamètre 4 cm, longueur 10 cm) en utilisant un gradient chloroforme-méthanol. On recueille ainsi 7 g de dérivé triprotégé, ce qui correspond à un rendement de 73%.

On hydrolyse alors les fonctions ester au moyen d'un mélange de triéthylamine, de pyridine et d'eau (20/20/60 en volume). On évapore ensuite les solvants et on purifie la $N_2$-méthoxyacétyl désoxy-2′

guanosine (composé n°3) par chromatographie sur colonne de silice silanisée en réalisant l'élution par un mélange d'eau et d'acétone (70/30 v/v). On obtient ainsi 3,4 g de produit, ce qui correspond à un rendement de 51%. On contrôle le produit par résonance magnétique nucléaire à 250 MHz et par spectrométrie de masse et l'on obtient les résultats suivants :

- (M - H) : pic moléculaire (m/e : 338 - 10%) ; guanine méthoxy-acétylée : (m/e : 222 - 31%).

EXEMPLE 4 : Préparation de ($N_6$-phénoxyacétyl)-désoxy-2' adénosine (composé n°4)

On sèche 1025 mg (4 mmol) de désoxyadénosine puis on les dissout dans 20 ml de pyridine distillée anhydre et on les introduit dans un ballon placé dans un bain d'eau glacée. On ajoute alors lentement 8 équivalents d'anhydride phénoxyacétique (9,5 g ; 32 mmol) dissous dans 20 ml de pyridine à 0° C. On laisse la réaction se poursuivre à la température ambiante pendant 90 min et une coloration jaunâtre apparaît progressivement. On forme ainsi le dérivé de nucléoside de formule (I) dans laquelle B représente le radical de formule (IV) dérivé de l'adénine, $R^1$ représente l'hydrogène, $R^2$ représente le radical phénoxy, $R^3$ et $R^4$ représentent le radical phénoxyacétyle et $R^5$ représente un atome d'hydrogène.

On détruit alors l'excès d'anhydride d'acide à 0° C par addition de 3 ml d'eau distillée, puis on dilue le milieu réactionnel par 100 ml de chloroforme. On lave la phase chloroformique 4 fois au moyen de 50 ml d'une solution aqueuse à 5% de bicarbonate de sodium, et on évapore le solvant, ce qui conduit à l'obtention d'un résidu jaune. On dissout celui-ci dans 100 ml de pyridine et, après avoir placé la solution dans un bain d'eau glacée, on ajoute 100 ml de soude 0,2 N à 0° C pour hydrolyser sélectivement les positions 3' et 5' de l'adénosine en 15 min. On neutralise alors la résine échangeuse de cations Dowex 50W-X8 sous forme pyridinium utilisée dans l'exemple 1. On filtre et on rince la résine puis on évapore à sec le filtrat.

On obtient ainsi la ($N_6$-phénoxyacétyl)-désoxy-2' adénosine (composé n°4) que l'on purifie par chromatographie sur colonne de silice (diamètre 4 cm et longueur 10 cm) en utilisant un gradient de chloroforme-méthanol (100-0-96-4). On évapore alors les fractions contenant le produit recherché et l'on obtient ainsi 1010 mg d'une poudre blanchâtre, ce qui correspond à un rendement de 65%.

On caractérise alors le produit par chromatographie sur couche mince, par résonance magnétique nucléaire du proton à 250 MHz et par spectrométrie de masse. Les résultats obtenus sont les suivants :

- $R_F$ : 0,66 avec le mélange de migration chloroforme-méthanol (80/20 en volume),
- résonance magnétique nucléaire du proton à 250 MHz : $^1$H-RMN (pyridine $d_5$) : 2,7-3,3 (m, 2H, $H_2$, $H_{2''}$; 4,1-4,35 (m, 2H, $H_5H_{5''}$) ; 4,6 (m, $H_{4'}$) ; 5,25 (m, $H_{3'}$) ; 5,65 (s, 2H, $CH_2$) ; 7,0 (m, $H_{1'}$) ; 6,9-7,4 (m, 5H, $C_6H_5$) ; 8,75 et 9,05 (s, $H_2$ et $H_8$).
- spectrométrie de masse : (M+H) : pic moléculaire (m/e : 386-16%) ; adénine phénoxy acétylée (m/e : 270 - 66%).

EXEMPLE 5 : Préparation de ($N_4$-isobutyryl)-désoxy-2' cytidine (composé n°5)

On sèche 4 mmol de désoxycytidine puis on les dissout dans 15 ml de pyridine anhydre et on introduit la solution dans un ballon placé dans un bain d'eau glacée. On ajoute alors lentement 6 équivalents (2,5 ml ; 24 mmol) de chlorure d'isobutyryle. On laisse la réaction se poursuivre à la température ambiante pendant 120 min et le milieu prend une teinte orangée. On forme ainsi le dérivé triprotégé du nucléoside de départ, soit le dérivé de formule (I) dans laquelle B représente le radical de formule (III) dérivé de la cytosine, $R_1$ et $R^2$ représentent le radical méthyle, $R^3$ et $R^4$ représentent le radical isobutyryle et $R^5$ représente un atome d'hydrogène.

On détruit l'excès de chlorure d'acide à 0° C par 2 ml d'eau bidistillée et on dilue le milieu réactionnel par 100 ml de chloroforme. On lave la phase chloroformique 4 fois par 50 ml de solution à 5% de bicarbonate de sodium pour éliminer l'acide isobutyrique formé, on sèche sur du sulfate de sodium et on évapore à sec. On obtient un résidu orange que l'on dissout dans 5 ml de pyridine refroidie à 0° C. On ajoute alors 100 ml de soude 0,2 N à 0° C et on laisse la réaction d'hydrolyse sélective des fonctions ester en 3' et 5' du désoxyribose se poursuivre pendant 30 min. On neutralise alors le milieu par la résine échangeuse d'ions Dowex 50W-X8 sous forme pyridinium utilisée dans l'exemple 1. On filtre ensuite et on rince la résine, puis on évapore à sec le filtrat.

On purifie par chromatographie sur colonne de silice (diamètre 4 cm ; L : 10 cm) en utilisant un gradient de chloroforme-méthanol (100-0 à 95-5). On évapore les solvants et l'on obtient ainsi 630 mg d'une poudre blanche constituée d'isobutyryl-désoxycytidine, ce qui correspond à un rendement de 50%.

On caractérise le produit par chromatographie sur couche mince, spectrométrie de masse et résonance magnétique nucléaire du proton à 250 MHz. Les résultats obtenus sont les suivants :

- $R_F$ : 0,55 avec le mélange de migration chloroforme-méthanol (80/20),
- spectrométrie de masse (M+H) : pic moléculaire (m/e = 298-11%), cytosine isobutyrylée (m/e = 182-100%)
- résonance magnétique nucléaire du proton à 250 MHz : $^1$H-RMN (méthanol $d_4$) : 1,2 (d, 6H, 2($CH_3$) de ib.), 2,15-2,6 (m, 2H, $H_{2'}$, $H_{2''}$), 2,7 (m, $H^{ib}$) ; 3,7-3,9 (m, 2H, $H_{5'}$, $H_{5''}$) ; 4,0 (m, $H_4$) ; 4,4 (m, $H_{3'}$) ; 6,25 (t, $H_{1'}$) ; 7,5 et 8,5 (d, $H_5$ et $H_6$).

EXEMPLE 6 : Préparation de ($N_4$-isobutyryl)(diméthoxy-4,4'-trityl)-5' désoxy-2' cytidine (composé n°6)

On sèche 2,5 mmol du composé n°5 par additions et évaporations successives de pyridine anhydre. On reprend par 25 ml de pyridine, on refroidit à 0°C et on ajoute 2,75 mmol (1,1 équivalent) de chlorure de diméthoxy-4,4' trityle dans 25 ml de pyridine, à 0°C. On laisse la réaction se poursuivre pendant 17 h à 5°C et on ajoute alors 2 ml de méthanol au milieu réactionnel. Après 30 min, on chasse le solvant à l'évaporateur rotatif et on reprend le résidu huileux par 100 ml d'acétate d'éthyle, et on lave par 3 fois 50 ml de solution aqueuse à 5% de $NaHCO_3$ et une fois 50 ml d'eau bidistillée. On sèche alors la phase organique sur sulfate de sodium et on la concentre. Par fractionnements sur colonne de gel de silice, on isole le composé n°6 ainsi obtenu qui répond à la formule (I) dans laquelle B est le radical de formule (III) dérivé de la cytosine, $R^1$ et $R^2$ sont des radicaux méthyle, $R^3$ est le radical diméthoxy-4,4' trityle, $R^4$ est un atome d'hydrogène et $R^5$ est un atome d'hydrogène.

Les caractéristiques physico-chimiques de ce composé et le rendement de la réaction sont donnés dans le tableau 1 annexé.

EXEMPLE 7 : préparation de( $N_6$-phénoxyacétyl)-(diméthoxy-4,4' trityl)-5' désoxy-2' adénosine (composé n°7)

On suit le même mode opératoire que dans l'exemple 6, mais en utilisant 2,5 mmol du composé n°4 au lieu du composé n°5 et l'on produit ainsi le composé n°7 : ($N_6$-phénoxyacétyl)-(diméthoxy-4,4' trityl)-5' désoxy-2' adénosine, soit le composé répondant à la formule (I) dans laquelle B est le radical dérivé de l'adénine de formule (IV), $R^1$ est un atome d'hydrogène, $R^2$ est le radical phénoxy, $R^3$ est le radical diméthoxy-4,4'-trityle, $R^4$ est un atome d'hydrogène et $R^5$ est un atome d'hydrogène.

Le rendement de la réaction et les caractéristiques physico-chimiques de ce composé sont données dans le tableau I annexé.

EXEMPLE 8 : Préparation du ($N_2$-méthoxyacétyl)-(diméthoxy-4,4' trityl)-5' désoxy-2' guanosine (composé n°8)

On suit le même mode opératoire que dans l'exemple 6, mais en utilisant 2,5 mmol du composé n°3 au lieu du composé n°5. On obtient ainsi le composé n°8 répondant à la formule (I) dans laquelle B est le radical de formule (II) dérivé de la guanine, $R^1$ est un atome d'hydrogène, $R^2$ est le radical méthoxy, $R^3$ est le radical diméthoxy-4,4'-trityle, $R^4$ est un atome d'hydrogène et $R^5$ est un atome d'hydrogène.

Le rendement de la réaction et les caractéristiques physico-chimiques de ce composé sont donnés dans le tableau 1 annexé.

Dans ce tableau, les lettres entre parenthèses indiquent la multiplicité du pic avec s=singulet, d=doublet, t=triplet ; q=quadruplet et m=multiplet.

EXEMPLE 9 : Préparation du composé n°9

Cet exemple illustre la préparation d'un dérivé phosphorylé du composé n°8 destiné à la synthèse phosphotriester de polynucléotides.

On sèche 3 mmol du composé n°8 par ajout et évaporation de pyridine anhydre (3 fois 5 ml). On reprend le résidu dans 15 ml de pyridine et on ajoute 4,5 mmol de bistriazolidate de chloro-4 phénylphosphoryle dans 30 ml de dioxanne anhydre (le bistriazolidate de chloro-4 phénylphosphoryle a été obtenu en ajoutant 4,5 mmol de dichlorophosphate de chloro-4 phényle à une suspension de 9 mmol de triazole et 9,35 mmol de triéthylamine dans 30 ml de dioxanne). On laisse la réaction se poursuivre pendant 20 min et on la stoppe alors par addition de 6 ml d'un mélange $H_2O$-triéthylamine (1/1 en volume) puis on réduit le volume du milieu réactionnel à 5 ml par évaporation. On reprend dans 100 ml de chloroforme et on lave 3 fois avec 50 ml de solution aqueuse de $NaHCO_3$ puis avec 100 ml d'eau. On sèche la phase chloroformique sur sulfate de sodium, puis on l'évapore à sec. Le composé recherché est isolé par chromatographie

sur gel de silice. On contrôle alors le produit obtenu par spectrométrie de masse et par résonance magnétique nucléaire. Le rendement de la réaction et les résultats obtenus sont donnés dans le tableau 2 annexé.

Le composé n°9 ainsi obtenu est le dérivé de nucléoside de formule (I) dans laquelle B représente le radical dérivé de la guanine de formule (II), $R^1$ représente un atome d'hydrogène, $R^2$ représente le radical méthoxy, $R^3$ représente le radical diméthoxy-4,4′ trityle, $R^4$ représente le radical de formule (VI) et $R^5$ représente un atome d'hydrogène.

EXAMPLE 10 : Préparation du composé n°10

Dans cet exemple, on suit le même mode opératoire que dans l'exemple 9 pour préparer le dérivé phosphorylé destiné à la synthèse phosphotriester du composé n°7 en utilisant 2,5 mmol du composé n°7 au lieu de 2,5 mmol du composé n°8. On obtient ainsi le composé n°10 qui répond à la formule (I) dans laquelle B représente le radical de formule (IV) dérivé de l'adénine, $R^1$ représente un atome d'hydrogène, $R^2$ représente le radical phénoxy, $R^3$ représente le radical diméthoxy-4,4′ trityle, $R^4$ représente le radical de formule (VI) et $R^5$ représente un atome d'hydrogène.

On contrôle comme précédemment les caractéristiques du composé obtenu par spectrométrie de masse et par résonance magnétique, les résultats obtenus et le rendement de la réaction sont donnés également dans le tableau 2 annexé.

EXEMPLE 11 : Préparation du composé n°11

Dans cet exemple, on suit le même mode opératoire que dans l'exemple 9 pour préparer le dérivé phosphorylé du composé n°6 destiné à la synthèse phosphotriester en utilisant 3 mmol du composé n°6 au lieu de 3 mmol du composé n°8.

On obtient ainsi le composé n°11 répondant à la formule (I) dans laquelle B représente le radical de formule (III) dérivé de la cytosine, $R^1$ et $R^2$ représentent le radical méthyle, $R^3$ représente le radical diméthoxy-4,4′ trityle, $R^4$ représente le radical de formule (VI) et $R^5$ représente un atome d'hydrogène.

Comme dans les exemples 9 et 10, on contrôle le produit obtenu par spectrométrie de masse et par résonance magnétique nucléaire. Le rendement de la réaction et les résultats obtenus sont donnés dans le tableau 2 annexé.

EXEMPLE 12 : Préparation du composé n°12

Dans cet exemple, on prépare le dérivé phosphorylé du composé n°8 destiné à la synthèse phosphoramidite d'oligonucléotides.

On sèche 3 mmol de composé n°8 par co-évaporation de pyridine, de toluène et de tétrahydrofuranne (THF). On reprend le résidu dans 15 ml de THF en présence de 12 mmol de N,N,N-diisopropyléthylamine et on ajoute goutte à goutte en 2 min 6 mmol de -cyanoéthyl-monochloro N,N-diisopropylaminophosphoramidite. Après 5 min de réaction, on observe la formation d'un précipité de chlorhydrate de l'amine. On laisse la réaction se poursuivre pendant 35 min et on filtre le précipité en fin de réaction, puis on évapore à sec le filtrat et on le reprend dans 150 ml d'acétate d'éthyle. On lave par une solution aqueuse glacée à 10% de $Na_2CO_3$. On sèche ensuite la phase organique sur sulfate de sodium et on l'évapore à sec.

On purifie le composé obtenu par chromatographie basse pression sur une colonne MERCK "LOBAR" de taille B en utilisant pour l'élution un mélange $CH_2Cl_2$-hexane-triéthylamine (70/20/10 de en volume). On reprend le composé obtenu par un minimum de dichlorométhane ou d'acétate d'éthyle et on le précipite dans l'hexane à -80 °C. On analyse le produit par résonance magnétique nucléaire. Les résultats obtenus et le rendement de la réaction sont donnés dans le tableau 3 annexé.

On obtient ainsi le composé n°12 qui répond à la formule (I) dans laquelle B représente le radical de formule (II), dérivé de la guanine, $R^1$ représente un atome d'hydrogène, $R^2$ représente le radical méthoxy, $R^3$ représente le radical diméthoxy-4,4′ trityle, $R^4$ représente le radical de formule (VII) et $R^5$ représente un atome d'hydrogène.

EXEMPLE 13 : Préparation du composé n°13

Dans cet exemple, on prépare comme dans l'exemple 12 le dérivé phosphorylé du composé n°7 destiné à la synthèse phosphoramidite en utilisant 3 mmol du composé n°7 au lieu de 3 mmol du composé n°8. On analyse également le produit obtenu par résonance magnétique nucléaire. Le rendement de la

réaction et les résultats sont donnés dans le tableau 3 annexé.

On obtient ainsi le composé n°13 qui répond à la formule (I) dans laquelle B est le radical de formule (IV) dérivé de l'adénine, $R^1$ est un atome d'hydrogène, $R^2$ est le radical phénoxy, $R^3$ est le radical diméthoxy-4,4' trityle, $R^4$ est le radical de formule (VII) et $R^5$ est un atome d'hydrogène.

EXEMPLE 14 : Préparation du composé N°14

Dans cet exemple, on prépare comme dans l'exemple 12 le dérivé phosphorylé du composé n°6 destiné à la synthèse phosphoramidite en utilisant 3 mmol du composé n°6 au lieu de 3 mmol du composé n°8. On analyse également le produit obtenu par résonance magnétique nucléaire. Le rendement de la réaction et les résultats obtenus sont donnés dans le tableau 3 annexé.

On obtient ainsi le composé n°14 qui répond à la formule (I) dans laquelle B représente le radical de formule (III) dérivé de la cytosine, $R^1$ et $R^2$ représentent le radical méthyle, $R^3$ représente le radical diméthoxy-4,4' trityle, $R^4$ représente le radical de formule (VII) et $R^5$ représente un atome d'hydrogène.

EXEMPLE 15 : Préparation de la N6-phénoxyacétyl (diméthoxy-4,4' trityl)-5' désoxy-2' guanosine (composé n°15).

On suit le même mode opératoire que dans l'exemple 6, en utilisant 4 mmol du composé n°1 au lieu du composé n°5 et l'on produit ainsi le composé n°15, soit le composé répondant à la formule (I) dans laquelle B est le radical dérivé de la guanine de formule (II), R1 est un atome d'hydrogène, R2 est le radical phénoxy, R3 est le radical diméthoxy-4,4' trityle, R4 est un atome d'hydrogène, R5 est un atome d'hydrogène.

Le rendement de la réaction est de 70%. Le Rf de ce composé dans le mélange Chloroforme/Méthanol (90:10) vaut 0,40. Les déplacements chimiques des principaux protons de cette molécule dans le méthanol deutérié ont les valeurs suivantes : H8 . 8,07 ppm, H1' : 6,45 ppm (t), H3': 4,75 ppm (m), H CH3 de diméthoxy trityle : 3,86 ppm (s), H CH2 de phénoxyacétyle : 5,05 ppm (s).

EXEMPLE 16 : Préparation du composé n°16.

Dans cet exemple on prépare comme dans l'exemple 12 le dérivé phosphorylé du composé n°15, destiné à la synthèse phosphoramidite en utilisant 3 mmol du composé n°15 au lieu de 3 mmol du composé n°8. Le rendement en produit final est de 50%. Le produit est caractérisé par un doublet en RMN du 31P localisé à 146 et 146,2 ppm dans la pyridine deutériée. Les principaux pics en RMN du proton dans l'acétonitrile deutérié sont localisés à : 8,12 ppm (s, H8), 6,45 ppm (t, H1'), 5,05 ppm (s, CH2 phénoxyacétyle) et 4,88 ppm (m, H3'). Par spectrométrie de masse à l'aide d'une source d'ions FAB il est possible d'observer le pic moléculaire de ce produit pour une valeur m/e de 903.

On obtient ainsi le composé n°16 répondant à la formule (I) dans laquelle B est le radical dérivé de la guanine de formule (II), R1 est un atome d'hydrogène, R2 est le radical phénoxy, R3 est le radical diméthoxy-4,4' trityle, R4 est le radical phosphoré de formule (VII), R5 est un atome d'hydrogène.

Les exemples 17 à 19 qui suivent illustrent la préparation de mononucléotides complètement protégés destinés à la synthèse d'oligonucléotides selon la méthode H-phosphonate.

EXEMPLE 17 : Préparation du composé n°17.

Dans cet exemple, on prépare le composé n°17 destiné à la synthèse des oligonucléotides selon la méthode H-Phosphonate.

On sèche 5 mmol de composé n°7 par co-évaporation de 5 ml de dioxanne anhydre et on les reprend dans 15 ml de ce solvant et 5 ml de pyridine anhydre. On additionne alors 5 ml d'une solution 1,25 M de chloro-2 benzo-5,6-a) [dioxo-1,3 phosphor-2 inone-4] ou salicylchlorophosphite de formule :

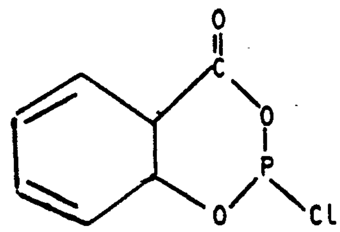

et on laisse la réaction se dérouler pendant 10 minutes à la température ambiante.

On additionne alors 0,5 ml d'eau et on laisse évoluer l'hydrolyse pendant 10 minutes. On verse alors ce mélange dans 250 ml de solution aqueuse molaire d'acétate de triéthylammonium et l'on extrait le produit désiré avec 2 fois 250 ml de chloroforme. On sèche la phase organique avec du sulfate de sodium anhydre et on la concentre à l'évaporateur rotatif. On purifie le résidu ainsi obtenu par chromatographie liquide haute performance sur une colonne de gel de silice (200×40 mm). On élue le produit avec des solutions à concentration croissante de méthanol dans un mélange à 2% de triéthylamine dans le chloroforme (0% de méthanol : 250 ml ; 1% de méthanol : 250 ml ; 2% de méthanol : 250 ml ; 3% de méthanol : 250 ml ; 5% de méthanol : 250 ml ; 7% de méthanol : 500 ml). On rassemble les fractions contenant le produit recherché et on évapore le solvant pour obtenir un produit se présentant sous la forme d'une mousse blanche. Le rendement en produit N°17 est de 55%. On analyse le produit par résonance magnétique nucléaire, les résultats obtenus sont regroupés dans le tableau IV.

On obtient ainsi le composé n°17 qui répond à la formule (I) dans laquelle B représente le radical de formule (IV), dérivé de l'adénine, $R_1$ représente un atome d'hydrogène, R2 représente le radical phénoxy, R3 représente le radical diméthoxy-4,4′ trityle, R4 représente le radical phosphoré de formule

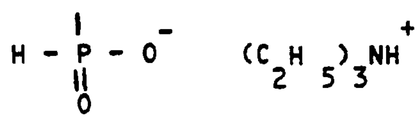

et R5 représente un atome d'hydrogène.

EXEMPLE 18 : Préparation du composé n°18.

Dans cet exemple, on prépare comme dans l'exemple 17 le dérivé phosphorylé du composé n°15 destiné à la synthèse d'oligonucléotides selon la méthode H-phosphonate en utilisant 5 mmol du composé n°15 au lieu de 5 mmol du composé n°7. Le rendement de la réaction est de 48% et on analyse le produit obtenu par résonance magnétique nucléaire. Les résultats obtenus sont présentés dans le tableau IV.

On obtient ainsi le composé n°18 qui répond à la formule (I) dans laquelle B représente le radical de formule (II), dérivé de la guanine, $R_1$ représente un atome d'hydrogène, R2 représente le radical phénoxy, R3 représente le radical dimétoxy-4,4′ trityle, R4 représente le radical phosphoré de formule

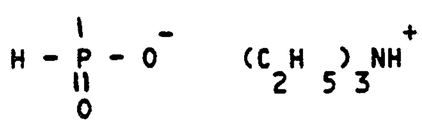

et R5 représente un atome d'hydrogène.

EXEMPLE 19 : Préparation du composé n°19.

Dans cet exemple, on prépare comme dans l'exemple 17 le dérivé phosphorylé du composé n°5 destiné à la synthèse d'oligonucléotides selon la méthode H-phosphonate en utilisant 5 mmol du composé n°5 au lieu de 5 mmol du composé n°7.

Le rendement de la réaction est de 62% et on analyse le produit obtenu par résonance magnétique nucléaire. Les résultats obtenus sont présentés dans le tableau IV.

On obtient ainsi le composé n°19 qui répond à la formule(I) dans laquelle B représente le radical de formule (III), dérivé de la cytosine, $R_1$ et R2 représentent le radical métyle, R3 représente le radical diméthoxy-4,4' trityle, R4 représente le radical phosphoré de formule

$$H-\underset{\underset{O}{\|}}{\overset{|}{P}}-O^{-}\qquad (C_2H_5)_3NH^{+}$$

et R5 représente un atome d'hydrogène.

EXEMPLE 20 :

Cet exemple illustre l'utilisation des composés n° 9, n°10 et n°11 pour la synthèse d'un oligonucléotide dont la séquence est la suivante :

d(AATTCAGATUTGATCAT) AGRE-AGRE

Dans cette séquence, A représente le nucléotide formé avec l'adénine, C représente le nucléotide formé avec la cytosine et G représente le nucléotide formé avec la guanine, T représente le nucléotide formé avec la thymine et U le nucléotide formé avec l'uracile.

Pour effectuer cette synthèse, on utilise les composés n°9, n°10 et n°11 comme synthons correspondant respectivement à la guanine, l'adénine, et à la cytosine et des synthons correspondant à la thymine et à l'uracile. Ceux-ci sont obtenus à partir des nucléosides correspondants en protégeant les fonctions hydroxyle en 5' et en 3' respectivement par le radical diméthoxy-4,4' trityle et le radical de formule VI) en utilisant le même mode opératoire que dans les exemples 6 à 11.

On effectue la synthèse au moyen d'un automate Biosearch "SAM ONE" en utilisant :

- 50 mg, soit environ 1,5 à 3 mol, du support comportant l'extrémité 5' de la chaîne (Pierce "Controlled Pore Glass"),
- 25 mg par cycle de condensation des dérivés de nucléosides obtenus dans les exemples 9 à 11 et des synthons de thymidine et de désoxy-2' uridine, ce qui représente environ 8 à 15 équivalents, et
- 25 mg par cycle de condensation, soit 2 équivalents par rapport au nucléoside, d'un agent d'activation constitué par du chlorure de mésitylène sulfonyle.

Les étapes composant chaque cycle de condensation sont les suivantes :

- détritylation : acide trichloracétique à 2 % dans $CH_2Cl_2$ pendant 2 min,
- lavage : $CH_3CN$ pendant 1 min,
- séchage : $CH_3CN$ anhydre pendant 6 min,
- condensation : dérivé de nucléoside monomère et chlorure de mésitylène sulfonyle dans le mélange $CH_3CN$/méthyl-1 imidazole (85/15 en volume pendant 15 min, et
- lavage : $CH_3CN$ pendant 6 min.

A la fin des cycles de condensation, on obtient donc un gel de silice comportant l'oligonucléotide lié de façon covalente. On transfère alors le tout dans une ampoule en pyrex, on ajoute de l'ammoniaque à 28%, et on la laisse pendant 8 h à la température ambiante. On libère ainsi l'oligonucléotide du support en éliminant également les groupes protecteurs faisant l'objet de l'invention.

On prélève alors le surnageant et on rince la silice avec 3 fois 1 ml d'eau bidistillée. On évapore le solvant et on reprend le résidu dans 0,5 ml d'eau, puis on le fractionne sur une colonne de Séphadex G 25 (diamètre 1 cm, H 7 cm), on recueille les fractions qui présentent une absorption dans l'ultraviolet à 254 nm et on les lyophilise.

On vérifie par marquage au phosphore 32 au moyen de la T4-polynucléotide kinase et par électrophorèse sur gel de polyacrylamide la bonne longueur du fragment d'ADN synthétique obtenu. Le découpage de la bande correspondante et l'élution du composé permettent de purifier le produit en vue d'une utilisation biologique.

On prépare de la même façon les oligonucléotides dont les séquences sont les suivantes :

- d(AATTCAGAUCTGATCAT),
- d(AATTCAGUTCTGATCAT),
- d(AATTCAUATCTGATCAT), et
- d(CGATGATCAGATCTG).

On obtient également de bons résultats et une élimination des groupes protecteurs dans des conditions douces, dans l'ammoniaque à température ordinaire.

EXEMPLE 21

Dans cet exemple, on utilise les composés n°12, n°13 et n°14 pour synthétiser des homopolymères de 15 nucléotides de longueur en utilisant la synthèse phosphoramidite.

On réalise les synthèses à l'aide du même automate Biosearch "SAM ONE" que dans l'exemple 20 et on utilise les quantités de réactif suivantes :

- 50 mg, soit de 1,5 à 3 mol du support utilisé dans l'exemple 15 comportant l'extrémité 3' de la chaîne polynucléotidique,
- 20 mg des composés 12, 13, ou 14 par cycle de condensation (20 à 25 équivalents), et
- 15 mg par cycle de condensation (2 équivalents par rapport au nucléotide) d'un agent d'activation constitué par le paranitrophényl-5 tétrazole.

Chaque cycle de condensation comporte les étapes suivantes :

- détritylation : acide trichloracétique à 2 % dans $CH_2Cl_2$ pendant 1 min 30,
- lavage : $CH_3CN$ pendant 1 min,
- séchage : $CH_3CN$ anhydre/diméthylformamide anhydre (90/10) pendant 3 min,
- condensation : dérivé de nucléoside + agent d'activation dans le mélange $CH_3CN$/diméthylformamide (90/10) pendant 3 min,
- oxydation : iode à 0,45% dans tétrahydrofuranne/eau/lutidine (89,5/10/0,5) pendant 1 min,
- masquage des fonctions hydroxyle n'ayant pas réagi pour stopper l'élongation des chaînes incomplètes : mélange d'anhydride acétique et de méthyl-1 imidazole dans $CH_3CN$ anhydre pendant 2 min,
- lavage : $CH_3CN$ pendant 3 min.

Après 14 cycles de condensation réalisés avec le même composé (composés n° 12, n°13 ou n°14), on transfère le support comportant le produit synthétisé dans une ampoule de pyrex et on ajoute 2 ml d'ammoniaque à 28%. On maintient les ampoules pendant 8 h à la température ambiante, ce qui permet d'éliminer les groupes protecteurs faisant l'objet de l'invention.

On prélève alors le surnageant et on rince la silice avec 3 fois 1 ml d'eau bidistillée, puis on chasse le solvant à l'évaporateur rotatif. On reprend le résidu brut dans 0,5 ml d'eau et on le purifie par chromatographie sur gel de Séphadex G 25. On recueille les fractions absorbant à 254 nm et on analyse leur contenu par électrophorèse sur gel de polyacrylamide après marquage au phosphore 32 par T4-polynucléotide kinase.

On contrôle ainsi que les 3 homopolymères synthétisés ont la longueur voulue et qu'ils correspondent respectivement à $d(A_{15})$, $d(C_{15})$ et $d(G_{15})$.

EXEMPLE 22 : Préparation d'un oligonucléotide par la méthode H-phosphonate.

Cet exemple illustre l'utilisation des composés n°17, n°18 et n°19 pour la synthèse d'un oligonucléotide dont la séquence est la suivante :

5' d(ATGATCTACT) 3'

Dans cette séquence, A, G, C et T représentent les mêmes nucléotides que dans la séquence de l'exemple 20.

Pour effectuer l'assemblage, on utilise les composés n°17, n°18 et n°19 comme synthons correspondant respectivement à l'adénine, la guanine et la cytosine et un synthon correspondant à la thymine. Ce dernier est obtenu à partir de la thymidine en protégeant la fonction 5' par un groupement diméthoxy-4,4' trityle et la fonction 3' par le groupement phosphoré de formule

$$H-\overset{|}{\underset{\overset{\|}{O}}{P}}-O^{-} \qquad (C_2H_5)_3NH^{+}$$

en utilisant le même mode opératoire que celui rapporté précédemment pour le dérivé H-phosphonate de l'adénine.

On effectue l'assemblage au moyen d'un automate Biosearch "SAM ONE" en utilisant :

- une colonne préconditionnée par le constructeur comprenant le support greffé avec 1 micromole de thymidine protégée en 5' par un groupement diméthoxy-4,4' trityle,
- 8 mg par cycle de condensation des dérivés de nucléosides n°17, n°18 et n°19 et du dérivé correspondant de la thymidine. Ce qui représente environ 10 équivalents molaires, et
- 6 microlitres par cycle de condensation soit 50 équivalents molaire de chlorure de triméthyl acétyle, utilisé comme agent d'activation.

Les étapes composant chaque cycle de condensation sont les suivantes :

- acide trichloracétique à 2% dans le dichlorométhane, 3ml en 1 minute,
- lavage à l' acétonitrile : 3 ml,
- séchage avec un mélange pyridine/acétonitrile 1:1 / 3 ml,
- condensation : dérivé du nucléoside et agent d'activation dans 2 ml de mélange pyridine/acétonitrile en 1 minute,
- lavage par 3 ml de mélange de pyridine et d'acétonitrile,
- lavage par 3 ml d'acétonitrile.

A la fin des cycles de condensation, on procède à l'oxydation des phosphores internucléotidiques par passage d'une solution d'iode à 2% dans un mélange pyridine/eau 98/2 (3ml). On effectue ensuite un lavage par le mélange de pyridine et d'acétonirile (5ml), par l'acétonitrile (3ml) et l'on procède à la détritylation comme il est décrit ci-dessus.

On obtient alors un gel de silice comportant le polynucléotide lié de façon covalente. On transfère le tout dans une ampoule en pyrex, on ajoute 2ml d'ammoniaque à 28% et on la laisse pendant 2 heures à la température ordinaire. On libère ainsi l'oligonucléotide du support en éliminant également les groupements protecteurs faisant l'objet de l'invention. On prélève alors le surnageant, on rince la silice avec 3 fois 1 ml d'eau bidistillée, on évapore le solvant et on reprend le résidu par 0,5 ml d'eau. On le fractionne ensuite sur une colonne de Séphadex G25 (diamètre 1 cm, hauteur 7 cm), on réunit les fractions présentant une absorption dans l'ultraviolet à 254 nm et on les lyophilise.

On vérifie la bonne longueur du produit synthétisé par marquage radio-actif au phosphore 32 au moyen de la T4-polynucléotide kinase suivi d'une électrophorèse sur gel de polyacrylamide.

TABLEAU I

| Composé n° | Rendement | Spectrométrie RMN $^1H$ à 250 MHz δ en ppm dans $CD_3OD$ + TMS | | | | |
|---|---|---|---|---|---|---|
| | | $H_{1'}$ | $H_{3'}$ | $CH_3$ (trityle) | Base | Protection de la base |
| 6 | 74 % | 6,15(*t*) | 4,5 (*q*) | 3,7 (*s*) | $H_5$ = 7,4(*d*), $H_6$ = 8,3(*d*) | $CH_3$ (isobutyryle) = 1,15(*d.d*) |
| 7 | 51 % | 6,51(*t*) | 4,68(*m*) | 3,72(*d*) | $H_2$, $H_8$ = 8,46-8,57(*s*) | $CH_2$ (phénoxyacétyle) = 4,97(*s*) |
| 8 | 76 % | 6,33(*t*) | 4,57(*m*) | 3,72(*s*) | $H_8$ = 8,04(*s*) | $CH_2$ (méthoxyacétyle) = 4,13(*s*)<br>$CH_3$ (méthoxyacétyle) = 3,46(*s*) |

TABLEAU II

| Composé n° | Rendement | Spect. masse[a)] | RMN $^{31}P$[b)] | RMN $^{1}H$ | | | |
|---|---|---|---|---|---|---|---|
| | | | | Sucre | | Base | Protection amide |
| | | | | $H_1'$ | $H_3'$ | | |
| 9 | 30 % | 830 (2,3 %) | -5,0 | 6,36(*t*) | 5,19(*m*) | $H_8$(*s*) = 8,03 | $CH_2$ = 4,13(*s*)<br>$CH_3$ = 3,46(*s*) |
| 10 | 72 % | 876 (6,7 %) | -5,5 | 6,56(*t*) | 5,28(*m*) | $H_2$, $H_8$(*s*) = 8,53 et 8,42 | $CH_2$ = 5,01(*s*) |
| 11 | 67 % | 788 (2,7 %) | -5,3 | 6,22(*t*) | 5,20(*m*) | $H_5$(*d*) = 7,56<br>H (*d*) = 8,19 | $CH_3$ (isobutyryle) (*d,d*) = 1,46 |

Table II - a) Source FAB ions négatifs. Mesure de la masse du pic phosphodiester. b) Les déplacements chimiques sont indiqués en ppm par rapport à $H_3PO_4$ 85 % pris comme référence externe.

TABLEAU III

| Composé n° | Rendement | RMN $^{31}P$ | RMN $^{1}H$ à 250 MHz [a] $CD_3CN$ | | | |
|---|---|---|---|---|---|---|
| | | | Sucre | | Base | Amide |
| | | | $H_{1'}$ | $H_{3'}$ | | |
| 12 | 51 % | 155,3<br>155,2 | 6,26 | 4,69 | $H_8$ = 7,87 | $CH_2$ = 4,12<br>$CH_3$ = 3,45 |
| 13 | 53 % | 155,3<br>155,1 | 6,44 | 4,93 | $H_2$, $H_8$ = 8,28 ; 8,56 | $CH_2$ = 4,99 |
| 14 | 85 % | 155,3 | 6,14 | 4,60 | 8,14 ; 8,21 | $CH_3$ (isobutyryle) = 1,07 |

**<u>Tableau III</u> - a) Les composés se présentent sous la forme d'un mélange de deux diastéréoisomères et les spectres RMN $^{1}H$ sont difficilement interprétables.**

Tableau IV

| COMPOSE N° | RMN 31P (ppm) | RMN DU PROTON | | | |
|---|---|---|---|---|---|
| | | SUCRE | | BASE | PROTECTION |
| | | H1' | H3' | | |
| 17 | 3,46 | 6.95 m | 5.66 m | H8 et H2<br>8,81 s<br>8,75 s | 5,69 s |
| 18 | 2,26 | 6,63 t | 5.30 m | H8<br>9.40 s | 5,71 s |
| 19 | 3,62 | 6.77 t | 5.60 m | H6 et H5<br>8.53 d<br>7,61 d | 1.25 dd |

Spectres enregistrés dans la pyridine deutériée.

Composé n° 9

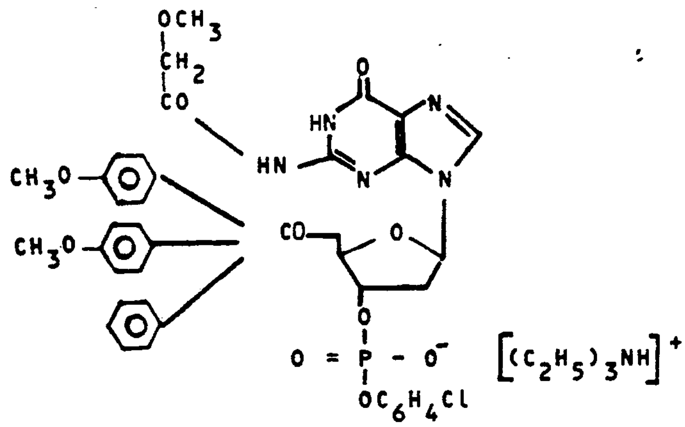

Composé n° 10

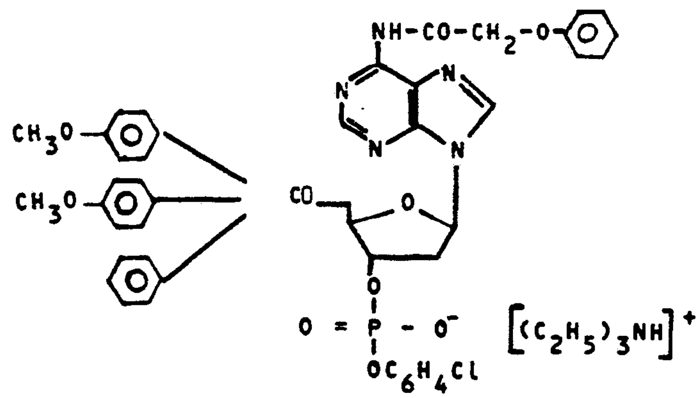

Composé n° 11

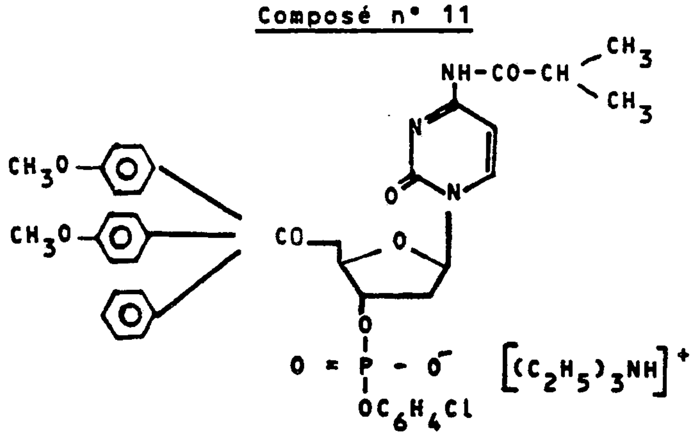

Composé n° 12

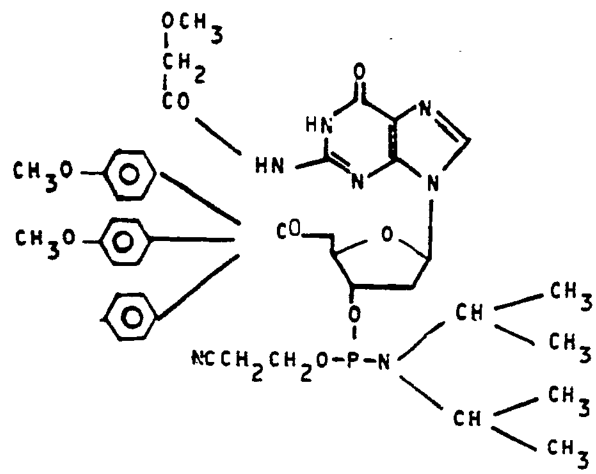

Composé n° 13

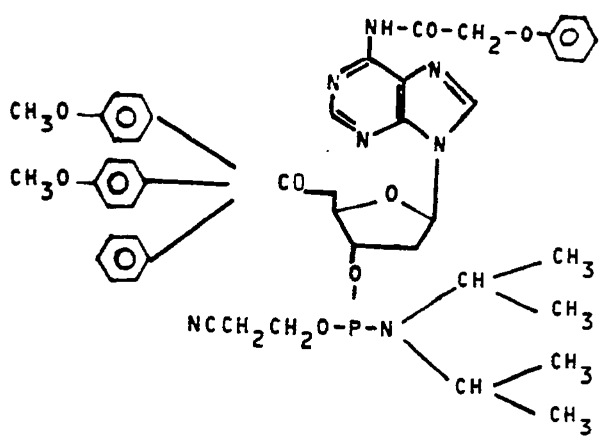

Composé n° 14

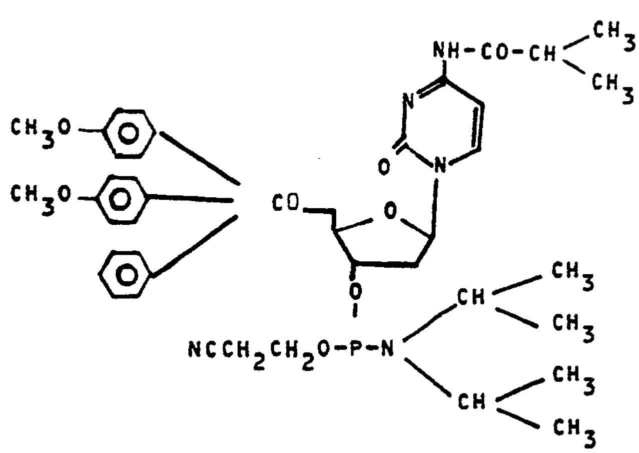

Composé n° 17

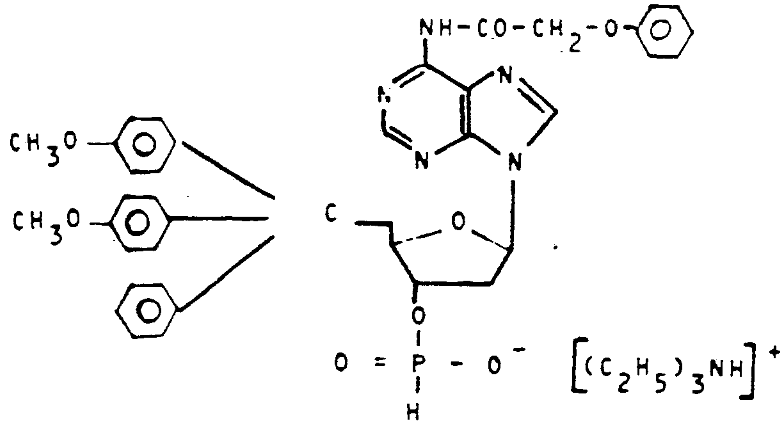

Composé n° 18

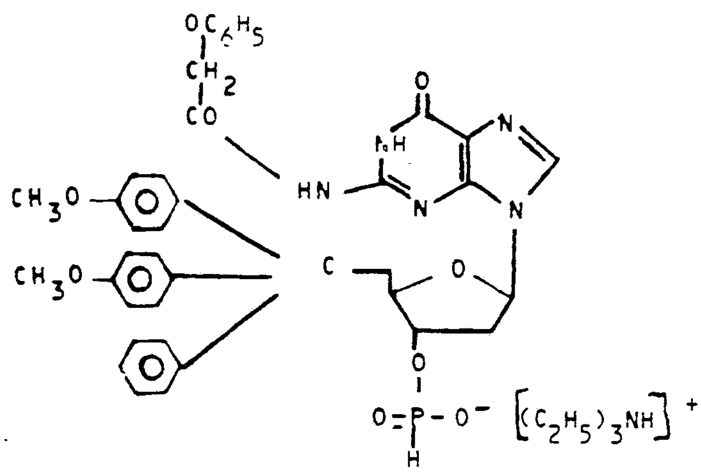

Composé n° 19

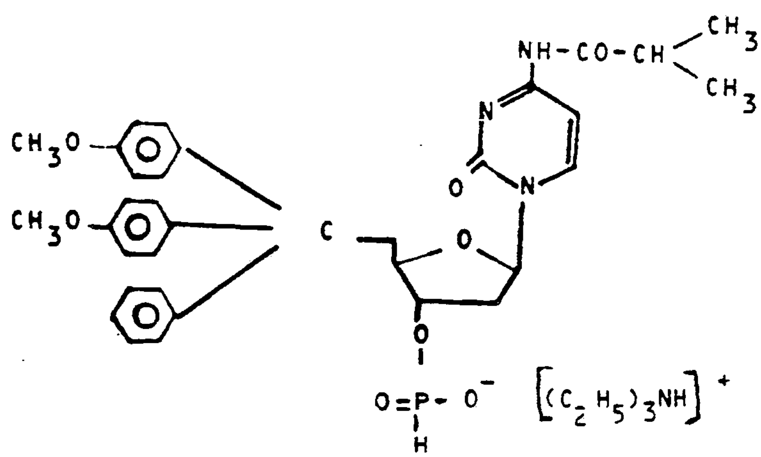

## Revendications

**Revendications pour les Etats contractants suivants : BE, CH, DE, FR, GB, GR, IT, LI, LU, NL, SE**

**1.** Dérivé de nucléoside répondant à la formule :

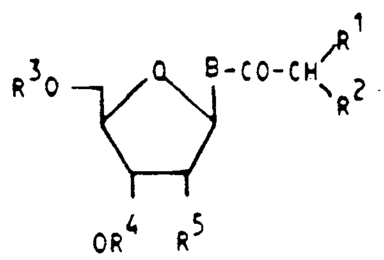

(I)

dans laquelle B représente un radical bivalent choisi parmi :

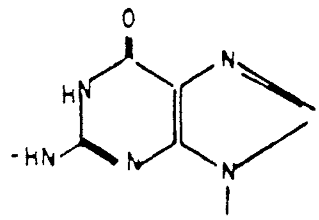

(II) ;

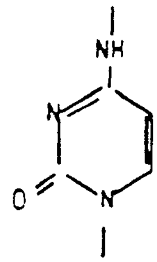

(III) ou

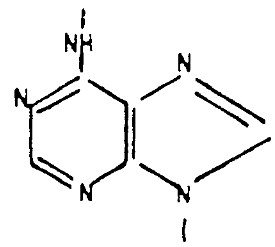

(IV)

relié au groupement CO par son groupement NH- exocyclique ;

- $R^1$ représente un atome d' hydrogène ou un radical méthyle ou éthyle ;
- $R^2$ représente un radical choisi dans le groupe constitué des radicaux méthoxy et phénoxy non substitué ou substitué par un ou des groupements choisis parmi $NO_2$, CN, méthoxy, éthoxy, phénoxy, Cl, F,

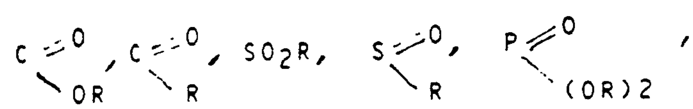

méthyle, éthyle ou phényle substitué ou non, SR avec R représentant un radical méthyle, éthyle ou phényle ;
- $R^3$ représente un atome d'hydrogène, un radical labile en milieu acide ou le radical de formule :

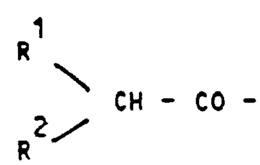

dans laquelle $R^1$ et $R^2$ ont la signification donnée cidessus,
- $R^4$ représente un atome d'hydrogène, un radical phosphoré ou le radical :

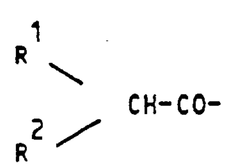

dans lequel $R^1$ et $R^2$ ont la signification donnée ci-dessus ; et
- $R^5$ représente un atome d'hydrogène ou le radical OH.

2. Dérivé de nucléoside selon la revendication 1, caractérisé en ce que le radical labile en milieu acide $R^3$ est choisi dans le groupe des radicaux trityle de formule :

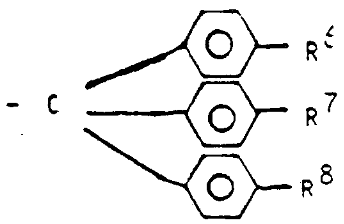

(V)

dans laquelle $R^6$ , $R^7$ et $R^8$ qui peuvent être identiques ou différents, représentent un atome d'hydrogène, un radical alkyle ou un radial alcoxy, des radicaux pixyle et des radicaux phényl-9 xanthényle.

3. Dérivé de nucléoside selon l'une quelconque des revendications 1 et 2, caractérisé en ce que le radical phosphoré $R^4$ est choisi dans le groupe des radicaux de formule :

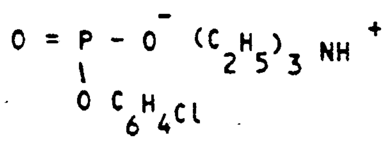

(VI)

de formule :

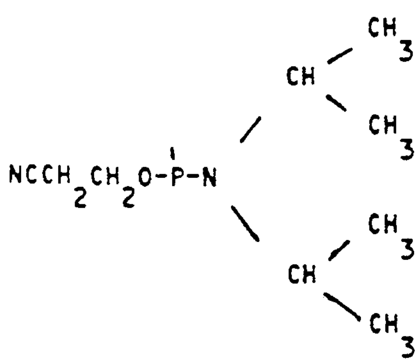

(VII)

ou de formule :

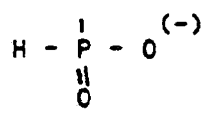

4. Dérivé de nucléoside selon la revendication 1, caractérisé en ce que B représente :

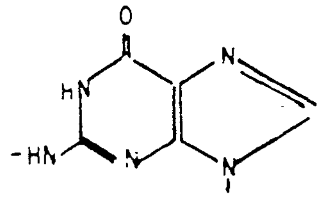 (II) ;

$R^1$ représente un atome d'hydrogène et $R^2$ est un radical choisi parmi :

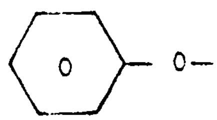 , 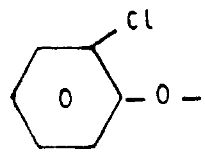 ou $CH_3O-$

5. Dérivé de nucléoside selon la revendication 1, caractérisé en ce que B représente :

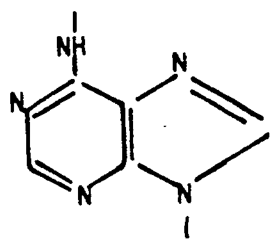 (IV)

$R^1$ représente un atome d'hydrogène et $R^2$ représente le radical phénoxy.

6. Dérivé de nucléoside selon l'une quelconque des revendications 1, 4 et 5, caractérisé en ce que $R^3$ et $R^4$ représentent un atome d'hydrogène.

7. Dérivé de nucléoside selon l'une quelconque des revendications 1, 4 et 5, caractérisé en ce que $R^3$ représente le radical :

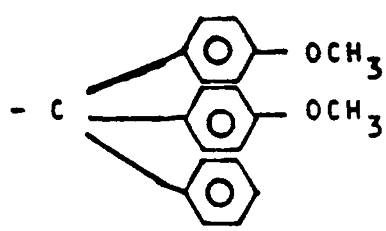

et $R^4$ représente un atome d'hydrogène.

8. Dérivé de nucléoside selon l'une quelconque des revendications 1 à 7, caractérisé en ce que $R^5$ représente un atome d'hydrogène.

9. Dérivé de nucléoside selon l'une quelconque des revendications 1, 4 et 5, caractérisé en ce que $R^3$ représente le radical :

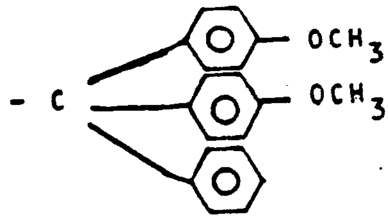

et $R^4$ représente le radical :

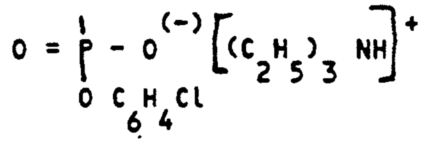 (VI)

**10.** Dérivé de nucléoside selon l'une quelconque des revendications 1, et 4 et 5, caractérisé en ce que $R^3$ représente le radical :

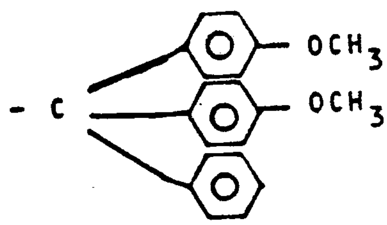

et $R^4$ représente le radical :

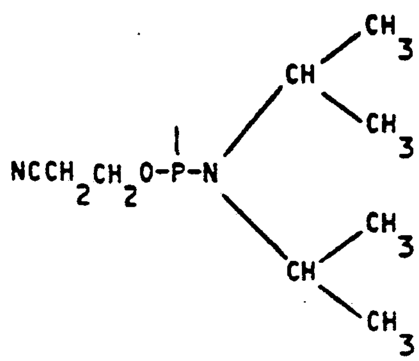 (VII)

**11.** Dérivé de nucléoside selon l'une quelconque des revendications 1, 4 et 5, caractérisé en ce que $R^3$ représente le radical :

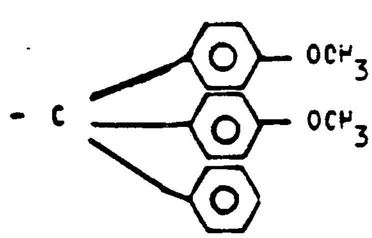

et $R^4$ représente le radical :

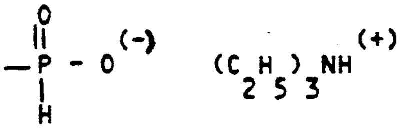

12. Dérivé de nucléoside selon l'une quelconque des revendications 9 à 11, caractérisé en ce que $R^5$ représente un atome d'hydrogène.

13. Procédé de synthèse d'oligonucléotides, caractérisé en ce qu'il comprend :

1° au moins un cycle de condensation dans lequel on condense sur un dérivé de nucléoside ou sur un oligonucléotide un dérivé de nucléoside de formule :

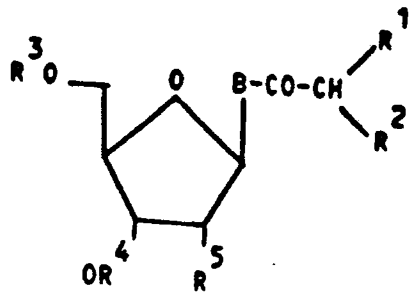

(I)

dans laquelle B représente un radical bivalent choisi parmi :

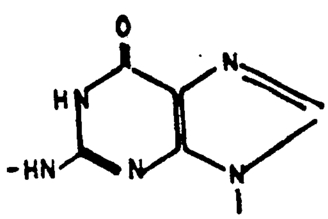 (II) ; 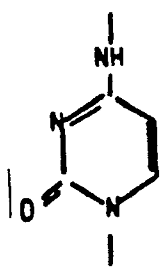 (III) ou 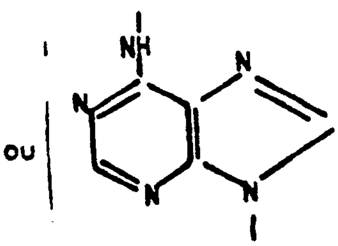 (IV)

relié au groupement CO par son groupement NH- exocyclique ;

- $R^1$ représente un atome d' hydrogène ou un radical méthyle ou éthyle ;
- $R^2$ représente un radical choisi dans le groupe constitué des radicaux méthoxy et phénoxy non substitué ou substitué par un ou des groupements choisis parmi $NO_2$, CN, méthoxy, éthoxy, phénoxy, Cl, F,

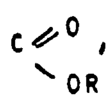, 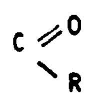, $SO_2R$, 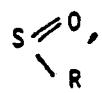, 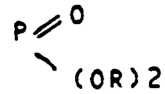,

méthyle, éthyle ou phényle substitué ou non, SR avec R représentant un radical méthyle, éthyle ou phényle ;
- $R^3$ représente un radical labile en milieu acide,
- $R^4$ représente un radical phosphoré, et
- $R^5$ représente un atome d'hydrogène : et

2°) une étape d'élimination du ou des groupes protecteurs de formule :

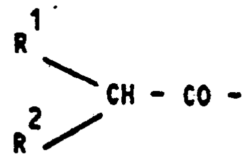

dans lesquelles $R^1$ et $R^2$ sont tels que définis ci-dessus.

**Revendications pour les Etats contractants suivants : ES, AT**

1. Procédé de préparation de dérivés de nucléosides de formule :

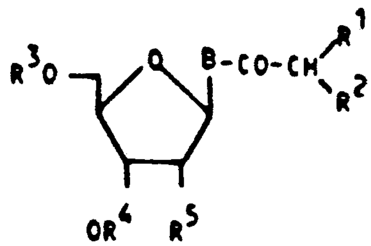 (I)

dans laquelle B représente un radical bivalent choisi parmi :

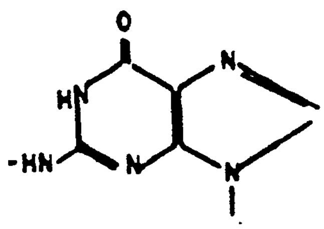 (II) ; 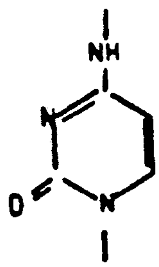 (III) ou 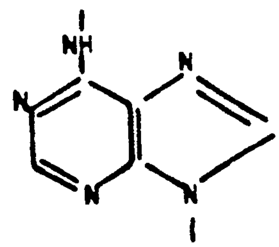 (IV)

relié au groupement CO par son groupement NH-exocyclique ;

- $R^1$ représente un atome d' hydrogène ou un radical méthyle ou éthyle ;
- $R^2$ représente un radical choisi dans le groupe constitué des radicaux méthoxy et phénoxy non substitué ou substitué par un ou des groupements choisis parmi $NO_2$, CN, méthoxy, éthoxy, phénoxy, Cl, F,

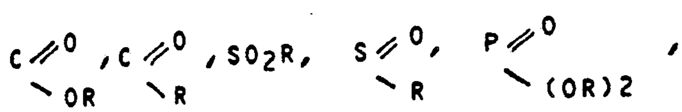

méthyle, éthyle ou phényle substitué ou non, SR avec R représentant un radical méthyle, éthyle ou phényle ;
- $R^3$ représente un atome d' hydrogène, un radical labile en milieu acide ou le radical de formule :

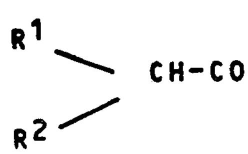

dans laquelle $R^1$ et $R^2$ ont la signification donnée ci-dessus,

- $R^4$ représente le radical :

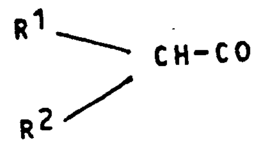

dans lequel $R^1$ et $R^2$ ont la signification donnée ci-dessus ; et
- $R^5$ représente un atome d'hydrogène, caractérisé en ce que l'on fait réagir un nucléoside choisi parmi les nucléosides de la guanine, de la cytosine et de l'adénine avec un chlorure d'acide de formule :

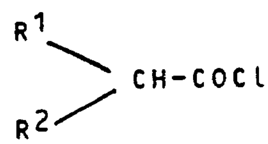

ou un anhydride d'acide de formule :

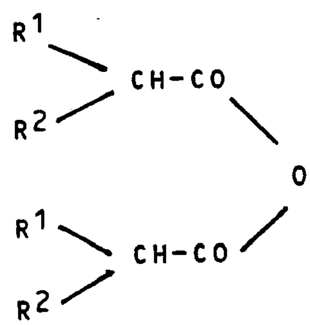

dans lesquelles $R^1$ et $R^2$ sont tels que définis ci-dessus.

**2.** Procédé de préparation d'un dérivé de nucléoside répondant à la formule :

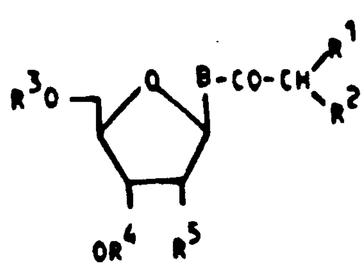

(I)

dans laquelle 8 représente un radical bivalent choisi parmi :

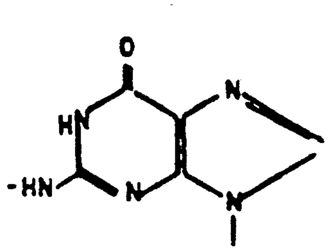 (II) ; 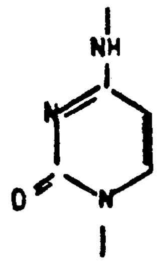 (III) ou 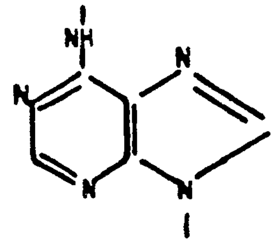 (IV)

relié au groupement CO par son groupement NH-exocyclique ;

- $R^1$ représente un atome d'hydrogène ou un radical méthyle ou éthyle ;
- $R^2$ représente un radical choisi dans le groupe constitué des radicaux méthoxy et phénoxy non substitué ou substitué par un ou des groupements choisis parmi $NO_2$, CN, méthoxy, éthoxy, phénoxy, Cl, F,

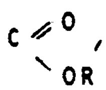 , 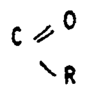 , $SO_2R$, 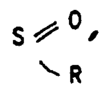 , 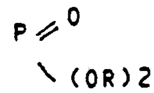 ,

méthyle, éthyle ou phényle substitué ou non, SR avec R représentant un radical méthyle, éthyle ou phényle ;
- $R^3$ représente un radical trityle de formule :

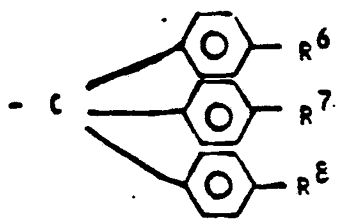 (V)

dans laquelle $R^6$, $R^7$ et $R^8$ qui peuvent être identiques ou différents, représentent un atome d' hydrogène, un radical alkyle ou un radical alcoxy,
- $R^4$ représente un radical phosphoré choisi parmi le radical de formule :

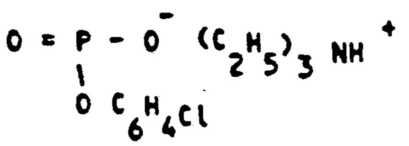 (VI)

le radical de formule :

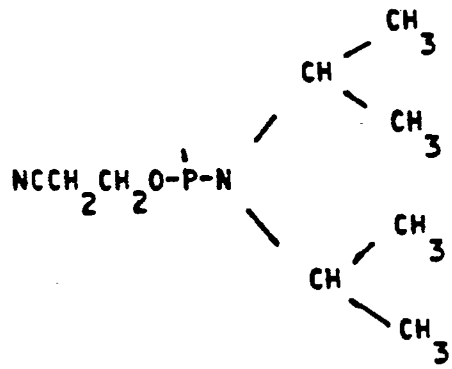

(VII)

- et le radical de formule :

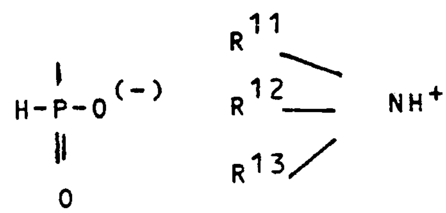

dans laquelle $R^{11}$, $R^{12}$ et $R^{13}$ qui peuvent être identiques ou différents, sont des radicaux alkyle , et

- $R^5$ représente un atome d' hydrogène,

caractérisé en ce qu'il comprend les étapes successives suivantes :

a) réaction d'un nucléoside choisi parmi les nucléosides de la guanine, de la cytosine et de l'adénine avec un chlorure d'acide de formule :

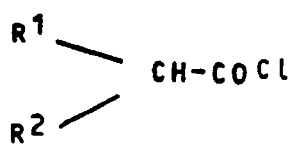

ou un anhydride d'acide de formule :

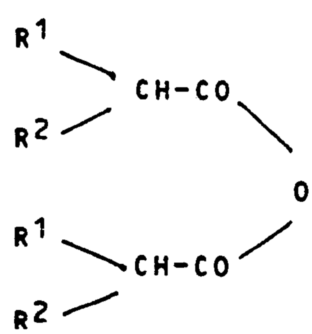

dans lesquelles $R^1$ et $R^2$ sont tels que définis cidessus, pour former un dérivé de nucléoside répondant à la formule (I) dans laquelle $R^3$ et $R^4$ représentent le radical de formule :

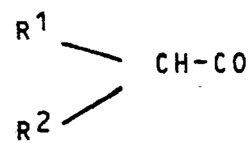

dans laquelle $R^1$ et $R^2$ sont tels que définis ci-dessus, et $R^5$ représente un atome d' hydrogène.

b) élimination des radicaux $R^3$ et $R^4$ du dérivé de nucléoside obtenu dans l'étape a) par hydrolyse sélective pour obtenir le dérivé de nucléoside de formule (I) dans laquelle $R^3$ et $R^4$ représentent un atome d'hydrogène ;

c) réaction du dérivé de nucléoside obtenu dans l'étape b) avec un chlorure de trityle de formule :

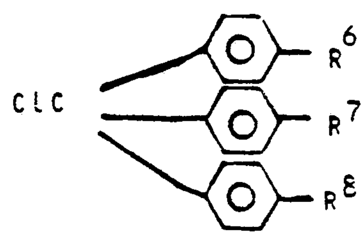

dans laquelle $R^6$, $R^7$ et $R^8$ qui peuvent être identiques ou différents, représentent un atome d' hydrogène, un radical alkyle ou un radical alcoxy, pour obtenir le dérivé de nucléoside de formule (I) dans laquelle $R^3$ représente un radical trityle ;

d) réaction du dérivé de nucléoside obtenu dans l'étape c) avec soit le bis-triazolidate de chloro-4-phénylphosphoryle, soit le $\beta$ -cyanoéthyl-monochloro N,N-diisopropylamine phosphoramidite, soit la chloro-2 benzo-(5, 6-a) dioxo-1,3 phosphor-2 inone-4 et un sel de trialkyl ammonium, pour obtenir le dérivé de nucléoside de formule (I) dans laquelle $R^3$ représente un radical trityle, $R^4$ représente un radical choisi parmi les radicaux de formule :

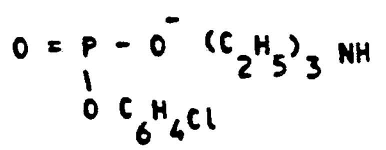 (VI)

de formule :

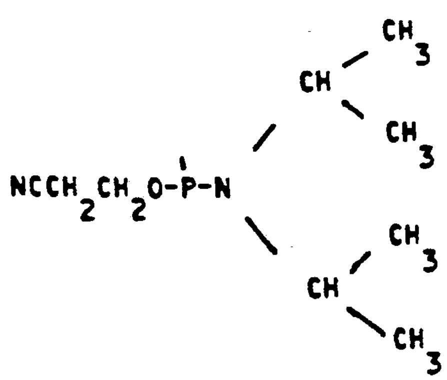 (VII)

ou de formule :

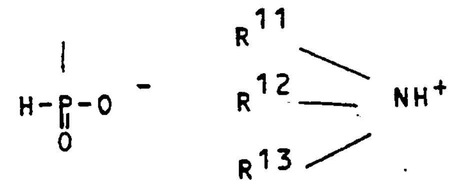

et $R^5$ représente l'hydrogène.

3. Procédé de synthèse d'oligonucléotides, caractérisé en ce qu'il comprend

1°) au moins un cycle de condensation dans lequel on condense sur un dérivé de nucléoside ou sur un oligonucléotide un dérivé de nucléoside de formule :

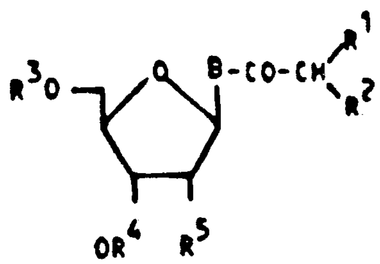 (I)

dans laquelle B représente un radical bivalent choisi parmi :

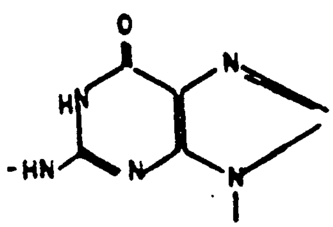 (II) ; 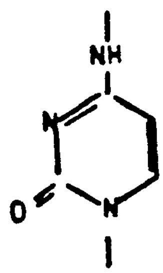 (III) ou 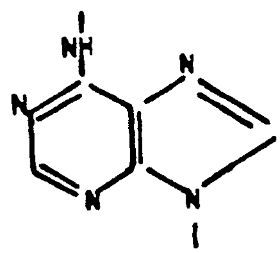 (IV)

relié au groupement CO par son groupement NH-exocyclique ;

- $R^1$ représente un atome d' hydrogène ou un radical méthyle ou éthyle ;
- $R^2$ représente un radical choisi dans le groupe constitué des radicaux méthoxy et phénoxy non substitué ou substitué par un ou des groupements choisis parmi $NO_2$, CN, méthoxy, éthoxy, phénoxy, Cl, F,

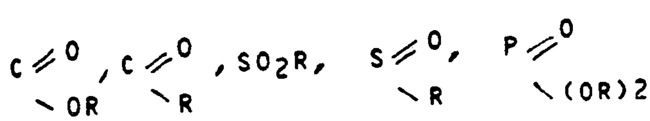

méthyle, éthyle ou phényle substitué ou non, SR avec R représentant un radical méthyle, éthyle ou phényle ;
- $R^3$ représente un radical labile en milieu acide,
- $R^4$ représente un radical phosphoré, et
- $R^5$ représente un atome d'hydrogène ; et

2°) une étape d'élimination du ou des groupes protecteurs de formule :

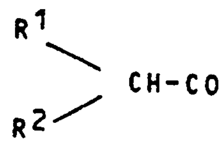

dans lesquelles $R^1$ et $R^2$ sont tels que définis ci-dessus.

4. Procédé selon la revendication 3, caractérisé en ce que le radical labile en milieu acide $R^3$ est choisi dans le groupe des radicaux trityle de formule :

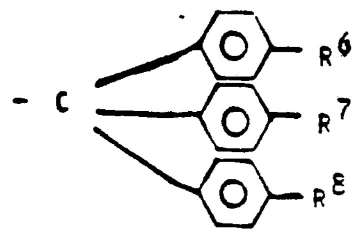

(V)

dans laquelle $R^6$, $R^7$ et $R^8$ qui peuvent être identiques ou différents, représentent un atome d' hydrogène, un radical alkyle ou un radical alcoxy, des radicaux pixyle et des radicaux phényl-9 xanthényle.

5. Procédé selon l'une quelconque des revendications 3 et 4, caractérisé en ce que le radical phosphoré $R^4$ est choisi dans le groupe des radicaux de formule :

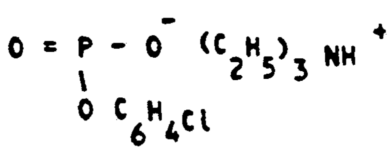

(VI)

de formule :

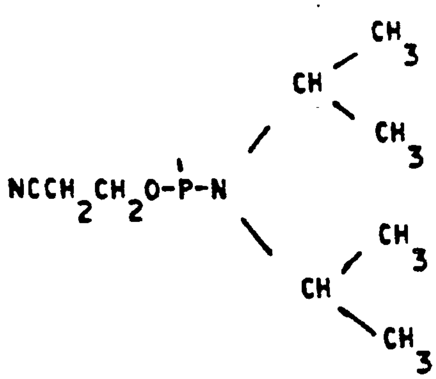

(VII)

ou de formule :

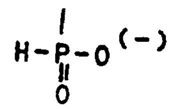 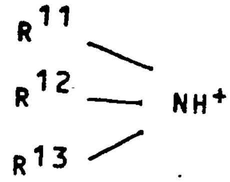

dans laquelle $R^{11}$, $R^{12}$ et $R^{13}$ qui peuvent être identiques ou différents, sont des radicaux alkyle.

6. Procédé selon l'une quelconque des revendications 3 à 5, caractérisé en ce que B représente :

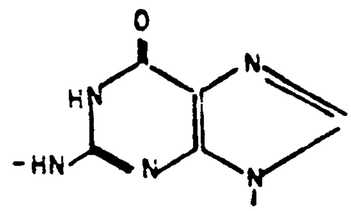 (II)

$R^1$ représente un atome d'hydrogène et $R^2$ est un radical choisi parmi :

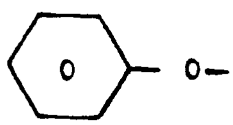 , 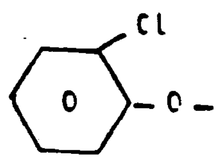 ou $CH_3O-$

7. Procédé selon l'une quelconque des revendications 3 à 5, caractérisé en ce que B représente :

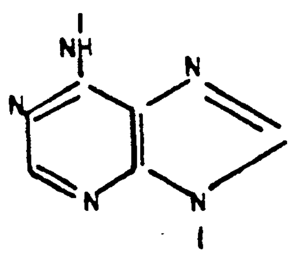 (IV)

$R^1$ représente un atome d' hydrogène et $R^2$ représente le radical phénoxy.

8. Procédé selon l'une quelconque des revendications 6 et 7, caractérisé en ce que $R^3$ représente le radical :

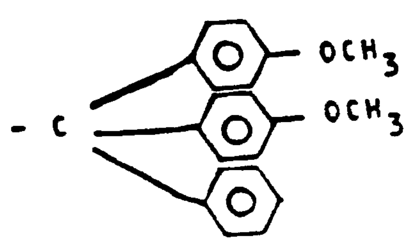

et $R^4$ représente le radical :

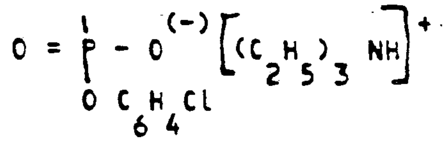
(VI)

**9.** Procédé selon l'une quelconque des revendications 6 et 7, caractérisé en ce que $R^3$ représente le radical :

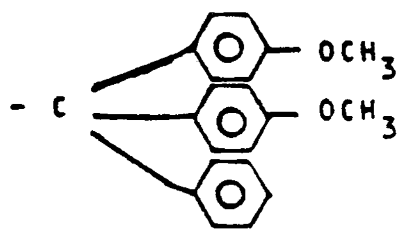

et $R^4$ représente le radical :

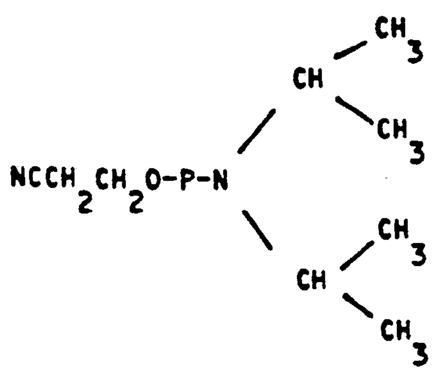
(VII)

**10.** Procédé selon l'une quelconque des revendications 6 et 7, caractérisé en ce que $R^3$ représente le radical de formule :

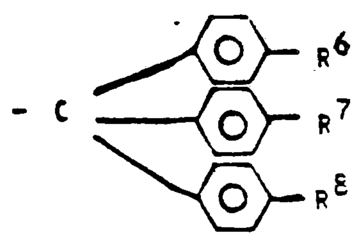
(V)

et $R^4$ représente le radical :

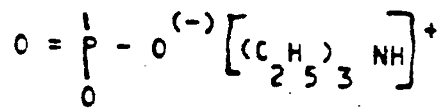

## Claims

**Claims for the following Contracting States : BE, CH, DE, FR, GB, GR, LI, LU, NL, SE**

1. Nucleoside derivative in accordance with formula:

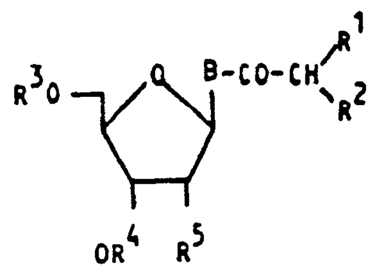

(I)

in which B represents a divalent radical chosen from among:

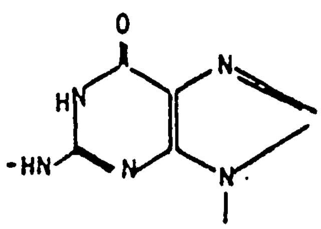 (II) ; 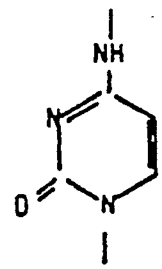 (III) or 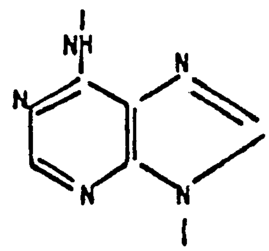 (IV)

connected to the CO group by its exocyclic NH group;
$R^1$ represents a hydrogen atom or a methyl or ethyl radical;
$R^2$ represents a radical chosen in the group constituted by methoxy and phenoxy radicals, which are unsubstituted or substituted by one or more groups chosen from among $NO_2$ , CN, methoxy, ethoxy, phenoxy, Cl , F

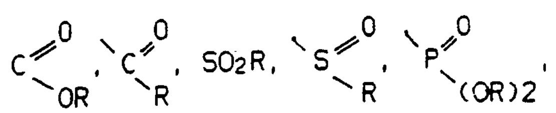

, , $SO_2R$, , ,

methyl , ethyl or phenyl , which may or may not be substituted, SR with R representing a methyl, ethyl or phenyl radical,
$R^3$ represents a hydrogen atom, an unstable radical in an acid medium or the radical of formula:

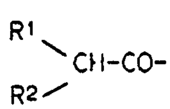

in which $R^1$ and $R^2$ have the meanings given hereinbefore;
$R^4$ represents a hydrogen atom, a phosphorus radical or the radical:

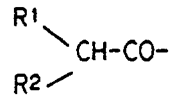

in which $R^1$ and $R^2$ have the above meaning; and $R^5$ represents a hydrogen atom or the OH radical.

**2.** Nucleoside derivative according to claim 1, characterized in that the unstable radical in the acid medium $R^3$ is chosen from the group of trityl radicals of formula:

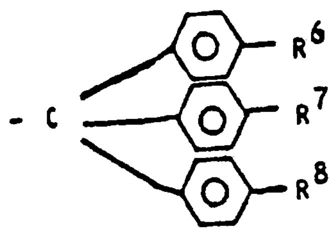

(V)

in which $R^6$ , $R^7$ and $R^8$, which can be the same or different, represent a hydrogen atom, an alkyl radical or an alkoxy radical, pixyl radicals and 9-phenyl-xanthenyl radicals.

**3.** Nucleoside derivative according to either of the claims 1 and 2, characterized in that the phosphorus radical R4 is chosen from the group of radicals of formula:

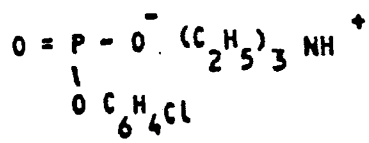

(VI)

of formula:

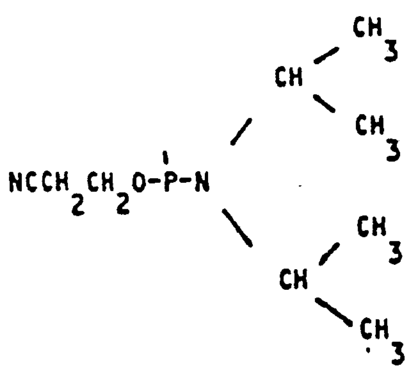

(VII)

or of formula:

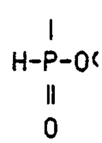

4. Nucleoside derivative according to claim 1, characterized in that B represents:

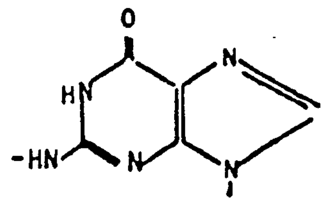

(II) ;

$R^1$ represents a hydrogen atom and $R^2$ is a radical chosen from among:

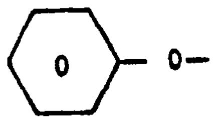

,

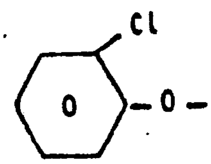

or $CH_3O-$

5. Nucleoside derivative according to claim 1, characterized in that B represents:

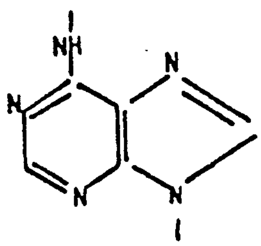

(IV)

$R^1$ represents a hydrogen atom and $R^2$ represents the phenoxy radical.

6. Nucleoside derivative according to any one of the claims 1, 4 and 5, characterized in that $R^3$ and $R^4$ represent a hydrogen radical.

7. Nucleoside derivative according to any one of the claims 1, 4 and 5, characterized in that $R^3$ represents the radical:

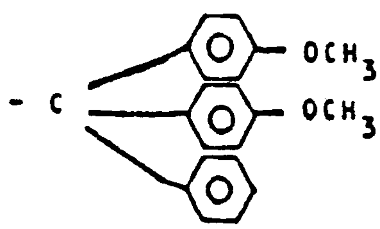

and R4 represents a hydrogen atom.

8. Nucleoside derivative according to any one of the claims 1 to 7, characterized in that $R^5$ represents a hydrogen atom.

9. Nucleoside derivative according to any one of the claims 1, 4 and 5, characterized in that $R^3$ represents the radical:

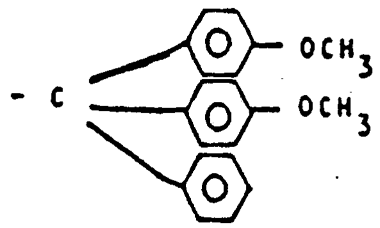

and R4 represents the radical:

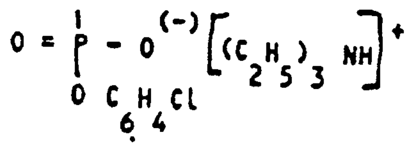 (VI)

**10.** Nucleoside derivative according to any one of the claims 1, 4 and 5, characterized in that $R^3$ represents the radical:

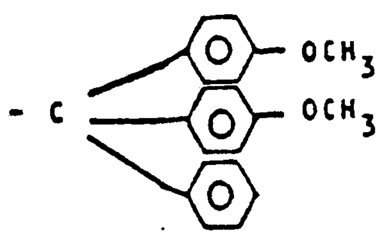

and $R^4$ represents the radical:

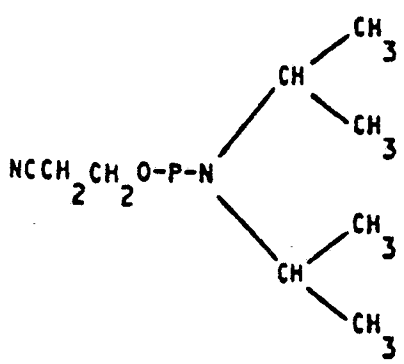 (VII)

**11.** Nucleoside derivative according to any one of the claims 1, 4 and 5, characterized in that $R^3$ represents the radical:

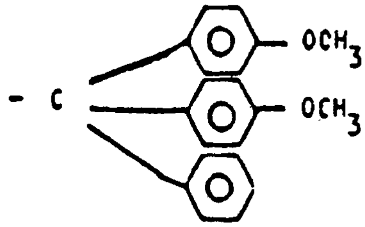

and $R^4$ represents:

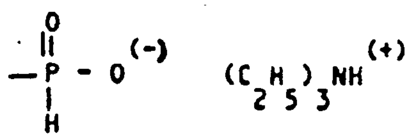

**12.** Nucleoside derivative according to any one of the claims 9 to 11, characterized in that $R^5$ represents a hydrogen atom.

**13.** Process for the synthesis of oligonucleotides, characterized in that it comprises:

1) at least one condensation cycle, in which on a nucleoside derivative or on an oligonucleotide is condensed a nucleoside derivative of formula:

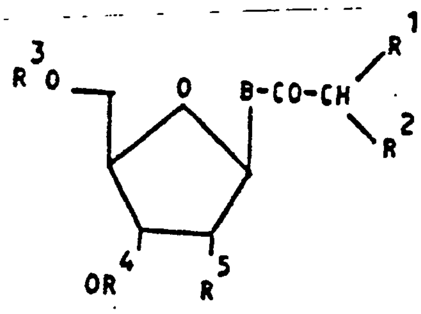

(I)

in which B represents a divalent radical chosen from among:

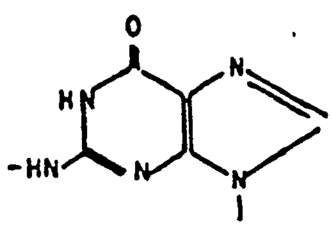 (II) ; 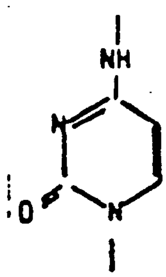 (III) or 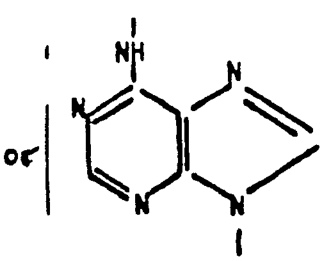 (IV)

connected to the CO group by its exocyclic NH group;

$R^1$ represents a hydrogen atom or a methyl or ethyl radical;

$R^2$ represents a radical chosen in the group constituted by methoxy and phenoxy radicals, which are unsubstituted or substituted by one or more groups chosen from among $NO_2$, CN, methoxy, ethoxy, phenoxy, Cl, F,

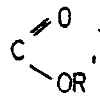, 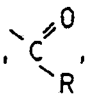, $SO_2R$, 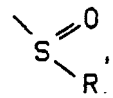, 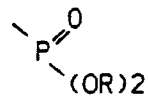,

methyl, ethyl or phenyl, which may or may not be substituted, SR with R representing a methyl, ethyl or phenyl radical,
$R^3$ an unstable radical in the acid medium;
$R^4$ represents a phosphorus radical; and
$R^5$ represents a hydrogen atom; and
2) a stage for the elimination of the protective group or groups of formula:

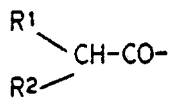

in which $R^1$ and $R^2$ are as defined hereinbefore.

**Claims for the following Contracting States : ES, AT**

**1.** Process for the preparation of nucleoside derivatives in accordance with the formula:

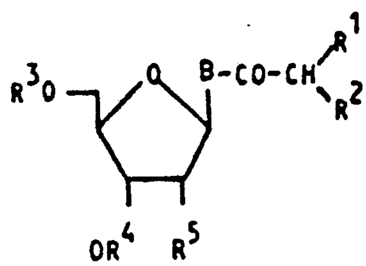 (I)

in which B represents a divalent radical chosen from among:

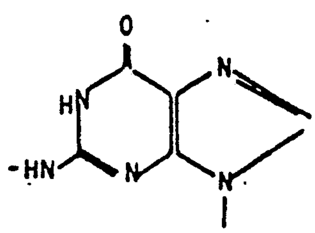 (II) ; 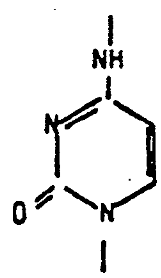 (III) or 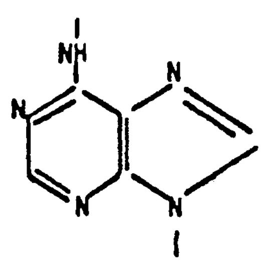 (IV)

connected to the CO group by its exocyclic NH group;
$R^1$ represents a hydrogen atom or a methyl or ethyl radical;
$R^2$ represents a radical chosen in the group constituted by methoxy and phenoxy radicals, which are unsubstituted or substituted by one or more groups chosen from among $NO_2$, CN, methoxy, ethoxy, phenoxy, Cl, F,

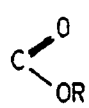, 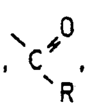, $SO_2R$, 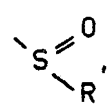, 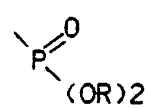,

methyl, ethyl or phenyl, which may or may not be substituted, SR with R representing a methyl, ethyl

or phenyl radical,
$R^3$ represents a hydrogen atom, an unstable radical in an acid medium or the radical of formula:

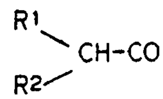

in which $R^1$ and $R^2$ have the meanings given hereinbefore,
$R^4$ represents the radical,

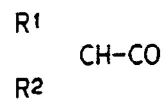

in which $R^1$ and $R^2$ have the meanings given hereinbefore and
$R^5$ represents a hydrogen atom,
characterized in that a nucleoside chosen from among the nucleosides of guanine, cytosine and adenine is reacted with an acid chloride of formula:

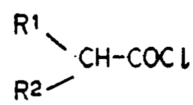

or an acid anhydride of formula:

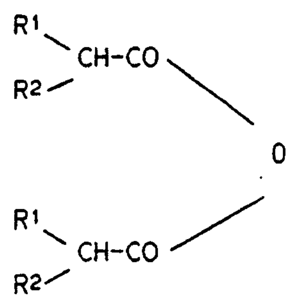

in which $R^1$ and $R^2$ are as defined hereinbefore.

2. Process for the preparation of a nucleoside derivative in accordance with the formula:

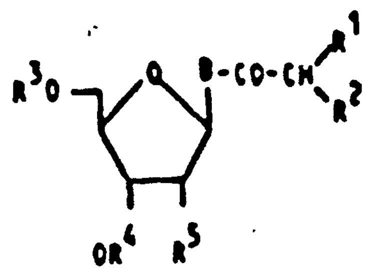

(1)

in which B represents a divalent radical chosen from among:

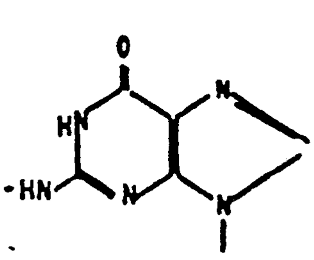 (II) ; 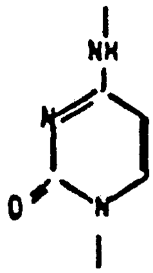 (III) ou 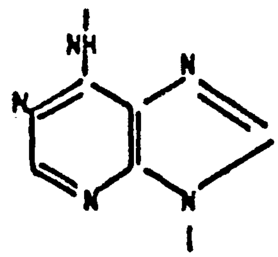 (IV)

connected to the CO group by its NH-exocyclic group;
$R^1$ represents a hydrogen atom or a methyl or ethyl radical;
$R^2$ represents a radical chosen in the group constituted by methoxy or phenoxy radicals, which are either unsubstituted or substituted by one or more groups chosen from among $NO_2$, CN, methoxy, ethoxy, phenoxy, Cl, F,

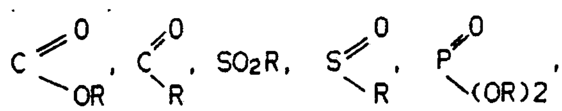

methyl, ethyl or phenyl, which may or may not be substituted, SR with R representing a methyl, ethyl or phenyl radical,
$R^3$ represents a trityl radical of formula:

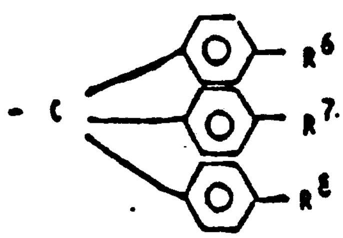 (V)

in which $R^6$, $R^7$ and $R^8$, which can be the same or different, represent a hydrogen atom, an alkyl radical or an alkoxy radical;
$R^4$ represents a phosphorus radical chosen from among the radical of formula:

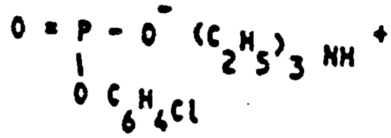 (VI)

the radical of formula:

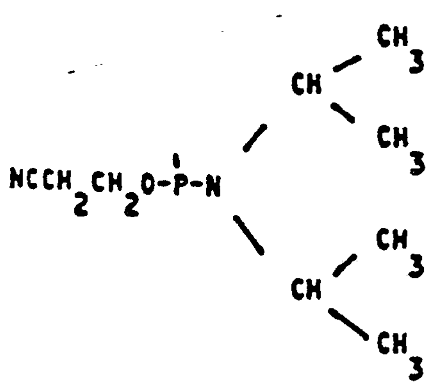 (VII)

and the radical of formula:

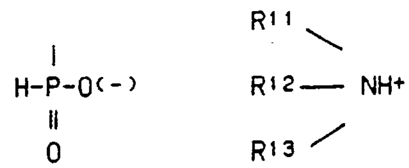

in which $R^{11}$, $R^{12}$ and $R^{13}$, which can be the same or different, are alkyl radicals and $R^5$ represents a hydrogen atom,
characterized in that it comprises the following successive stages:

a) reaction of a nucleoside chosen from among the nucleosides of guanine, cytosine and adenine with an acid chloride of formula:

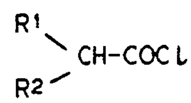

or an acid anhydride of formula:

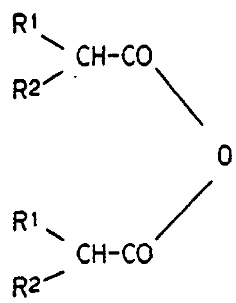

in which $R^1$ and $R^2$ are as defined hereinbefore, for forming a nucleoside derivative complying with formula (I), in which $R^3$ and $R^4$ represent the radical of formula:

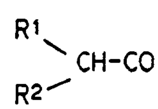

in which $R^{\cdot}$ and $R^2$ are as defined hereinbefore and $R^5$ represents a hydrogen atom,

b) elimination of the radicals $R^3$ and $R^4$ from the nucleoside derivative obtained in stage a) by selective hydrolysis in order to obtain the nucleoside derivative of formula (1), in which $R^3$ and $R^4$ represent a hydrogen atom,

c) reaction of the nucleoside derivative obtained in stage b) with a trityl chloride of formula:

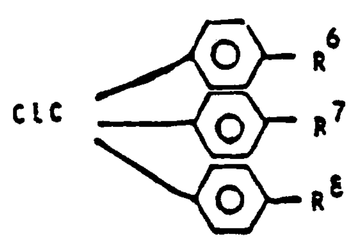

in which $R^6$, $R^7$ and $R^8$, which can be the same or different, represent a hydrogen atom, an alkyl

radical or an alkoxy radical, to obtain the nucleoside derivative of formula (I), in which $R^3$ represents a trityl radical,

d) reaction of the nucleoside derivative obtained in stage c) with either the chloro-4-phenyl phosphoryl bis-triazolidate, or the $\beta$-cyanoethyl-monochloro-N,N-diisopropylamine phosphoramidite, or the chloro-2-benzo-(5,6-a)-1,3-dioxo-2-phosphor-4-inone and a trialkyl ammonium salt, to obtain the nucleoside derivative of formula (I), in which $R^3$ represents a trityl radical, $R^4$ represents a radical chosen from among the radicals of formula:

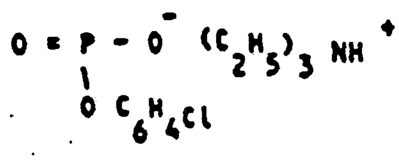

(VI)

of formula:

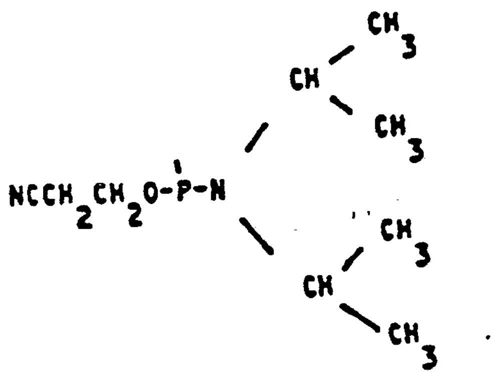

(VII)

or formula:

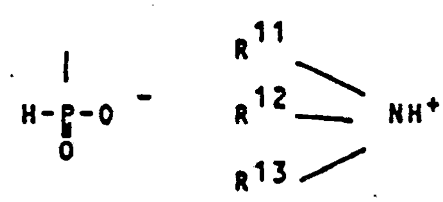

and $R^5$ represents hydrogen.

**3.** Process for the synthesis of oligonucleotides, characterized in that it comprises

1) at least one condensation cycle in which on a nucleoside derivative or an oligonucleotide is condensed a nucleoside derivative of formula:

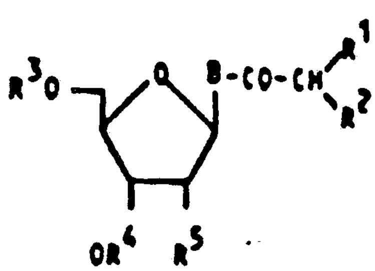

(I)

in which B represents a divalent radical chosen from among:

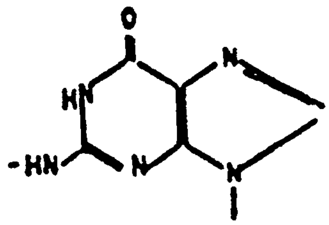 (II) ; 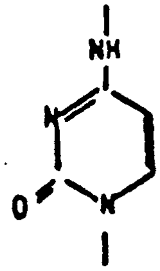 (III) ou 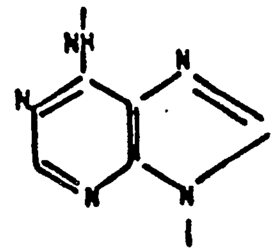 (IV)

connected to the CO group by its NH-exocyclic group;
$R^1$ represents a hydrogen atom or a methyl or ethyl radical;
$R^2$ represents a radical chosen from the group constituted by methoxy and phenoxy radicals, which are either unsubstituted or substituted by one of the groups chosen from among $NO_2$, CN, methoxy, ethoxy, phenoxy, Cl, F,

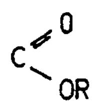 , 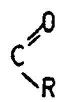 , $SO_2R$, 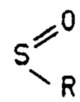 , 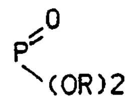 ,

methyl, ethyl or phenyl, which may or may not be substituted, SR with R representing a methyl, ethyl or phenyl radical,
$R^3$ represents a radical unstable in the acid medium;
$R^4$ represents a phosphorus radical; and
$R^5$ represents a hydrogen atom; and

2) a stage for eliminating the protective group or groups of formula:

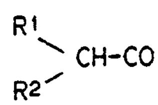

in which $R^1$ and $R^2$ are as defined hereinbefore.

4. Process according to claim 3, characterized in that the radical unstable in the acid medium $R^3$ is chosen in the group of trityl radicals of formula:

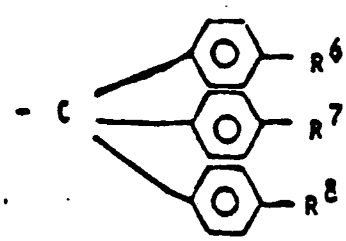 (V)

in which $R^6$, $R^7$ and $R^8$, which can be the same or different, represent a hydrogen atom, an alkyl radical or an alkoxy radical, pixyl radicals and 9-phenyl-xanthenyl radicals.

5. Process according to either of the claims 3 and 4, characterized in that the phosphorus radical $R^4$ is chosen from the group of radicals of formula:

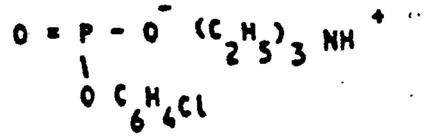 (VI)

of formula:

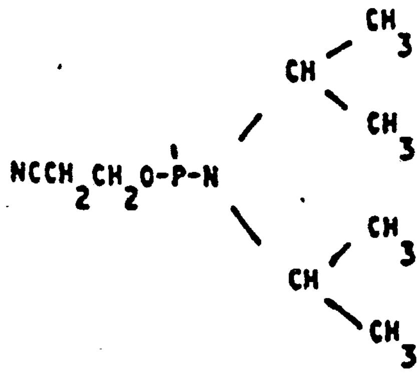 (VII)

or of formula:

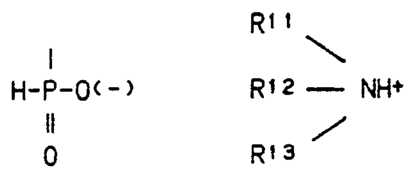

in which $R^{11}$, $R^{12}$ and $R^{13}$, which can be the same or different, are alkyl radicals.

6. Process according to any one of the claims 3 to 5, characterized in that B represents:

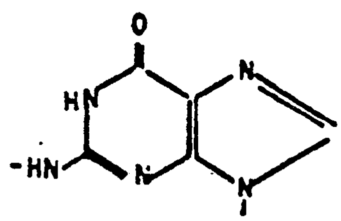 (II)

and $R^1$ represents a hydrogen atom and $R^2$ is a radical chosen from among:

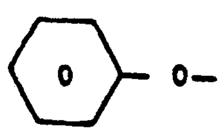 , 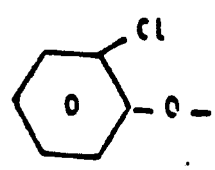 ou $CH_3O-$

7. Process according to any one of the claims 3 to 5, characterized in that B represents:

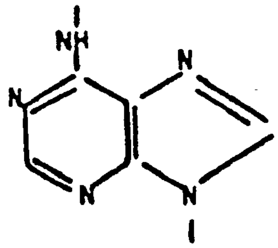

(IV)

and $R^1$ represents a hydrogen atom, $R^2$ representing the phenoxy radical.

**8.** Process according to one of the claims 6 and 7, characterized in that $R^3$ represents the radical:

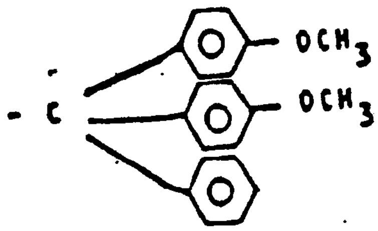

and $R^4$ represents the radical:

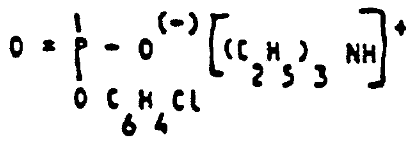

(VI)

**9.** Process according to any one of the claims 6 and 7, characterized in that $R^3$ represents the radical:

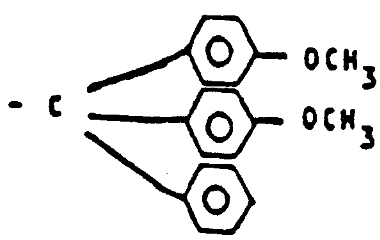

and $R^4$ represents the radical:

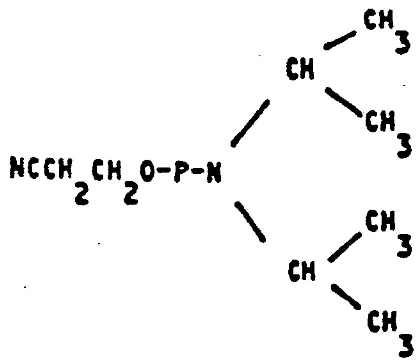

(VII)

**10.** Process according to any one of the claims 6 and 7, characterized in that $R^3$ represents the radical of formula:

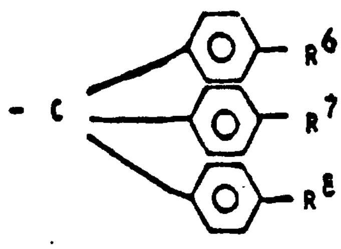

(V)

and $R^4$ represents the radical:

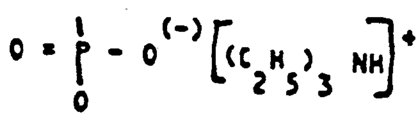

**Patentansprüche**

**Patentansprüche für folgende Vertragsstaaten : BE, CH, DE, FR, GB, GR, IT, LI, LU, NL, SE**

**1.** Nucleosidderivat der Formel

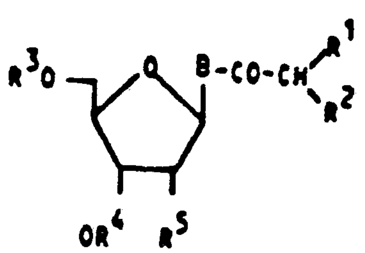

(I)

worin bedeuten:

B einen bivalenten Rest, ausgewählt aus der Gruppe:

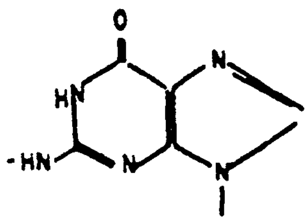 (II) ; 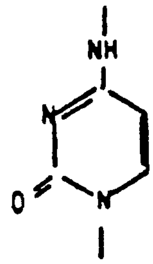 (III) oder 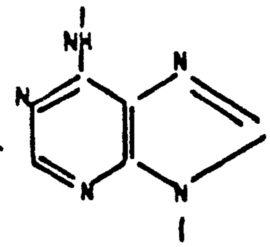 (IV)

der über seine exocyclische NH-Gruppe an die CO-Gruppe gebunden ist;

$R^1$ ein Wasserstoffatom oder einen Methyl- oder Ethylrest;

$R^2$ einen Rest, ausgewählt aus der Gruppe, die besteht aus den Resten Methoxy und Phenoxy, unsubstituiert oder substituiert durch eine oder mehrere Gruppen, ausgewählt aus $NO_2$, CN, Methoxy, Ethoxy, Phenoxy, Cl, F,

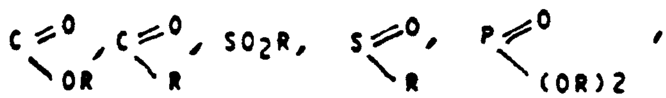

Methyl, Ethyl oder Phenyl, substituiert oder unsubstituiert, SR, worin R einen Mewthyl-, Ethyl- oder Phenylrest darstellt;

$R^3$ ein Wasserstoffatom, einen in saurem Milieu labilen Rest oder den Rest der Formel

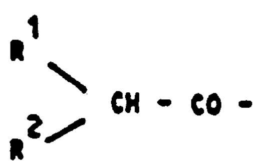

worin $R^1$ und $R^2$ die oben angegebenen Bedeutungen haben;

$R^4$ ein Wasserstoffatom, einen phosphorhaltigen Rest oder den Rest

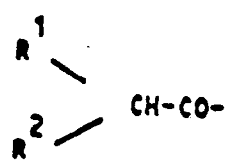

worin $R^1$ und $R^2$ die oben angegebenen Bedeutungen haben; und

$R^5$ ein Wasserstoffatom oder den OH-Rest.

2. Nucleosidderivat nach Anspruch 1, dadurch gekennzeichnet, daß der in saurem Milieu labile Rest $R^3$ ausgewählt wird aus der Gruppe der Tritylreste der Formel:

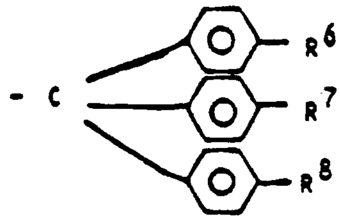 (V)

worin $R^6$, $R^7$ und $R^8$, die gleich oder verschieden sein können, ein Wasserstoffatom, einen Alkylrest oder einen Alkoxyrest darstellen, der Pixylreste und der 9-Phenyl-xanthenylreste.

**3.** Nucleosidderivat nach einem der Ansprüche 1 und 2, dadurch gekennzeichnet, daß der phosphorhaltige Rest $R^4$ ausgewählt wird aus der Gruppe der Reste der Formel

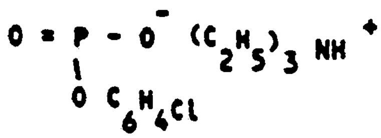 (VI)

der Formel:

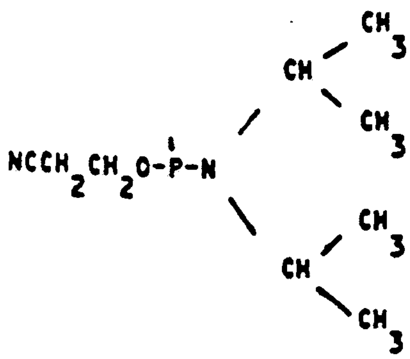 (VII)

oder der Formel:

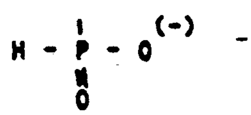

**4.** Nucleosidderivat nach Anspruch 1, dadurch gekennzeichnet, daß B steht für:

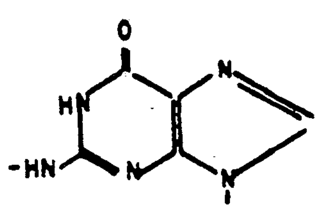 (II) ;

$R^1$ steht für ein Wasserstoffatom und

$R^2$ steht für einen Rest, der ausgewählt wird aus:

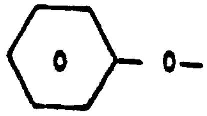 , 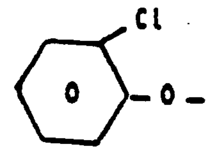 oder $CH_3O-$

5. Nucleosidderivat nach Anspruch 1, dadurch gekennzeichnet, daß B steht für

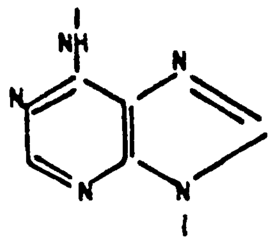 (IV)

$R^1$ steht für ein Wasserstoffatom und

$R^2$ steht für den Phenoxyrest.

6. Nucleosidderivat nach einem der Ansprüche 1, 4 und 5, dadurch gekennzeichnet, daß $R^3$ und $R^4$ ein Wasserstoffatom darstellen.

7. Nucleosidderivat nach einem der Ansprüche 1, 4 und 5, dadurch gekennzeichnet, daß $R^3$ steht für den Rest

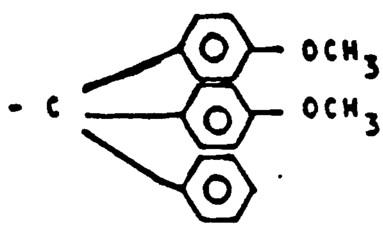

und $R^4$ steht für ein Wasserstoffatom.

8. Nucleosidderivat nach einem der Ansprüche 1 bis 7, dadurch gekennzeichnet, daß $R^5$ ein Wasserstoffatom darstellt.

9. Nucleosidderivat nach einem der Ansprüche 1, 4 und 5, dadurch gekennzeichnet, daß $R^3$ steht für den Rest

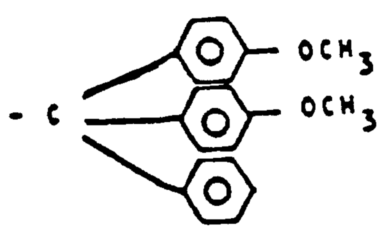

und $R^4$ steht für den Rest

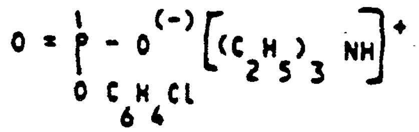 (VI)

**10.** Nucleosidderivat nach einem der Ansprüche 1, 4 und 5, dadurch gekennzeichnet, daß $R^3$ steht für den Rest:

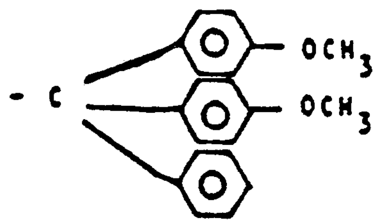

und $R^4$ steht für den Rest:

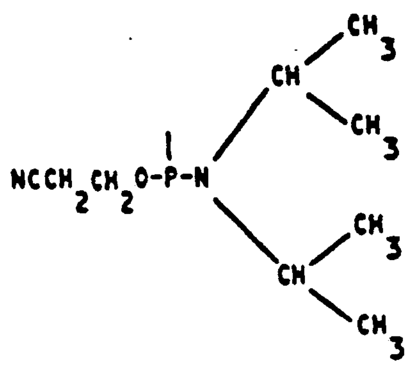 (VII)

**11.** Nucleosidderivat nach einem der Ansprüche 1, 4 und 5, dadurch gekennzeichnet, daß $R^3$ steht für den Rest:

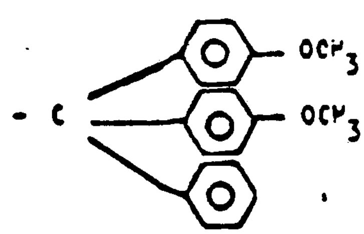

und $R^4$ steht für den Rest:

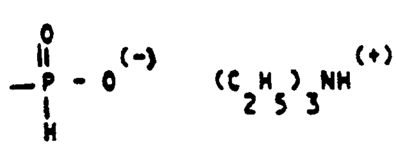

**12.** Nucleosidderivat nach einem der Ansprüche 9 bis 11, dadurch gekennzeichnet, daß $R^5$ ein Wasserstoffatom darstellt.

**13.** Verfahren zur Synthese von Oligonucleotiden, dadurch gekennzeichnet, daß es umfaßt

1) mindestens einen Kondensationscyclus, in dem man ein Nucleosidderivat der Formel

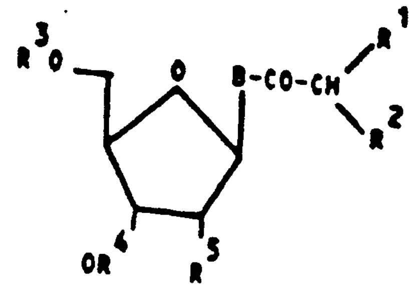

(I)

worin bedeuten:

B einen bivalenten Rest, der ausgewählt wird aus der Gruppe:

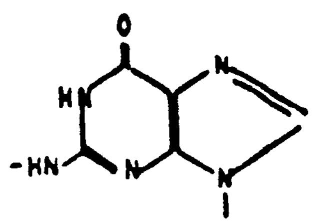 (II) ; 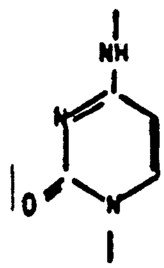 (III) oder 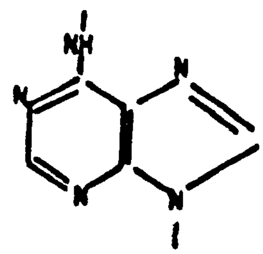 (IV)

der über seine exocyclische NH-Gruppe an die CO-Gruppe gebunden ist;

$R^1$ ein Wasserstoffatom-oder einen Methyl- oder Ethylrest;

$R^2$ einen Rest, ausgewählt aus der Gruppe, die besteht aus den Resten Methoxy und Phenoxy, unsubstituiert oder substituiert durch eine oder mehrere der Gruppen, ausgewählt aus $NO_2$, CN, Methoxy, Ethoxy, Phenoxy, Cl, F,

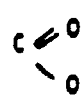 , 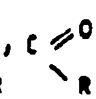 , $SO_2R$, 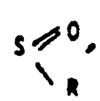 , 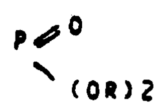 ,

Methyl, Ethyl oder Phenyl, substituiert oder unsubstituiert, SR, worin R für einen Methyl-, Ethyl- oder Phenylrest steht;

$R^3$ einen in saurem Milieu labilen Rest;

$R^4$ einen phosphorhaltigen Rest; und

$R^5$ ein Wasserstoffatom;

an ein Nucleosidderivat oder an ein Oligonucleotid ankondensiert; und

2) eine Stufe zur Eliminierung der Schutzgruppe(n) der Formel:

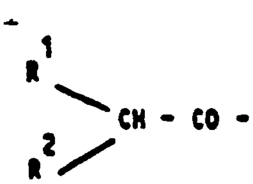

worin $R^1$ und $R^2$ wie oben definiert sind.

**Patentansprüche für folgende Vertragsstaaten : ES, AT**

**1.** Verfahren zur Herstellung von Nucleosidderivaten der Formel:

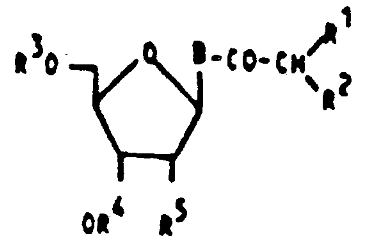

(I)

worin bedeuten:

B einen bivalenten Rest, ausgewählt aus der Gruppe:

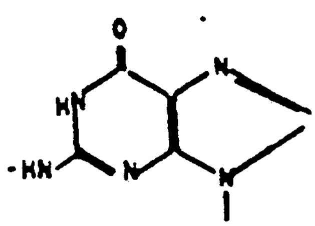

(II) ;

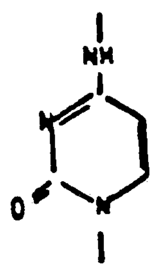

(III) oder

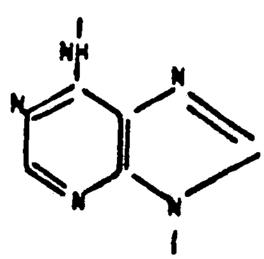

(IV)

der über seine exocyclische NH-Gruppe an die CO-Gruppe gebunden ist;
$R^1$ ein Wasserstoffatom oder einen Methyl- oder Ethylrest;
$R^2$ einen Rest, ausgewählt aus der Gruppe, die besteht aus den Resten Methoxy und Phenoxy, unsubstituiert oder substituiert durch eine oder mehrere der Gruppen, ausgewählt aus $NO_2$, CN, Methoxy, Ethoxy, Phenoxy, Cl, F,

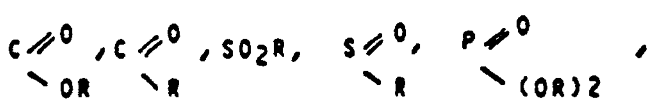

Methyl, Ethyl oder Phenyl, substituiert oder unsubstituiert, SR, worin R einen Methyl-, Ethyl- oder Phenylrest darstellt; $R^3$ ein Wasserstoffatom, einen in saurem Milieu labilen Rest oder den Rest der Formel

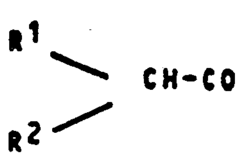

worin $R^1$ und $R^2$ die oben angegebenen Bedeutungen haben;
$R^4$ den Rest

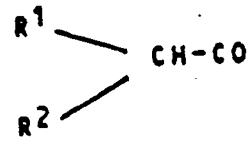

worin $R^1$ und $R^2$ die oben angegebenen Bedeutungen haben; und $R^5$ ein Wasserstoffatom,

dadurch gekennzeichnet, daß man ein Nucleosid, ausgewählt aus den Nucleosiden von Guanin, Cytosin und Adenin, mit einem Säurechlorid der Formel

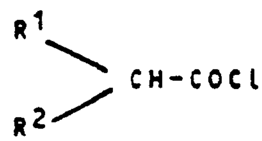

oder einem Säureanhydrid der Formel

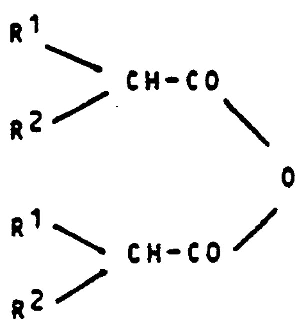

worin $R^1$ und $R^2$ die oben angegebenen Bedeutungen haben,

reagieren läßt.

2. Verfahren zur Herstellung eines Nucleosidderivats der Formel:

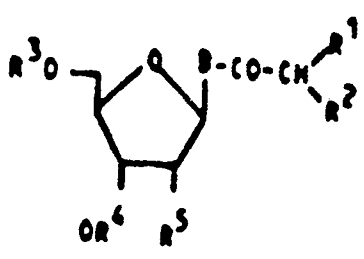

(I)

worin bedeuten:
B einen bivalenten Rest, ausgewählt aus der Gruppe:

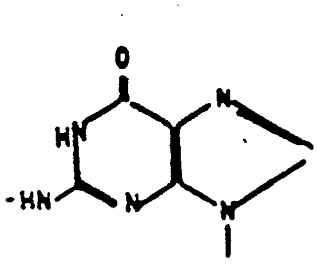 (II) ; 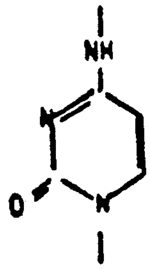 (III) oder 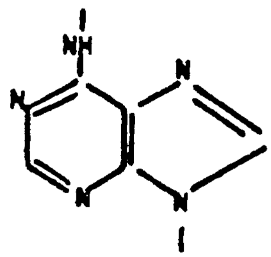 (IV)

der über seine exocyclische NH-Gruppe an die CO-Gruppe gebunden ist;
$R^1$ ein Wasserstoffatom oder einen Methyl- oder Ethylrest;
$R^2$ einen Rest, ausgewählt aus der Gruppe, die besteht aus den Resten Methoxy und Phenoxy, unsubstituiert oder substituiert durch eine oder mehrere der Gruppen, ausgewählt aus $NO_2$, CN, Methoxy, Ethoxy, Phenoxy, Cl, F,

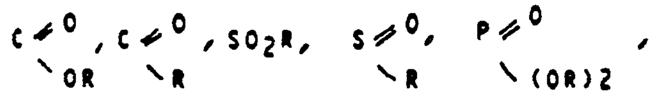

Methyl, Ethyl oder Phenyl, substituiert oder unsubstituiert, SR, worin R einen Methyl-, Ethyl- oder Phenylrest darstellt; $R^3$ einen Tritylrest der Formel

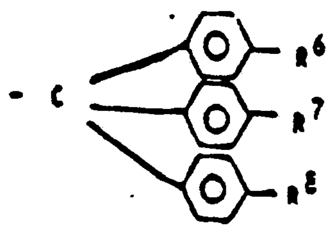 (V)

worin $R^6$, $R^7$ und $R^8$, die gleich oder verschieden sein können, ein Wasserstoffatom, einen Alkylrest oder einen Alkoxyrest darstellen,
$R^4$ einen phosphorhaltigen Rest, ausgewählt aus dem Rest der Formel:

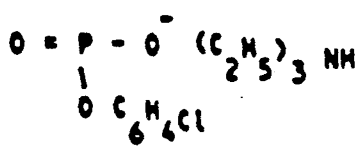 (VI)

dem Rest der Formel:

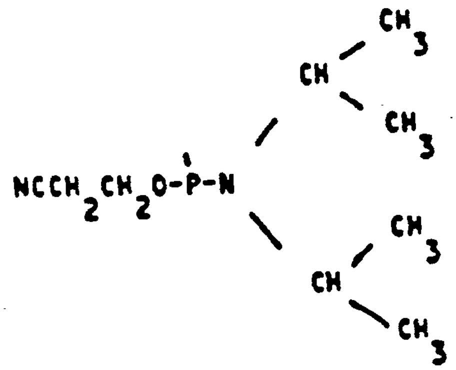 (VII)

und dem Rest der Formel:

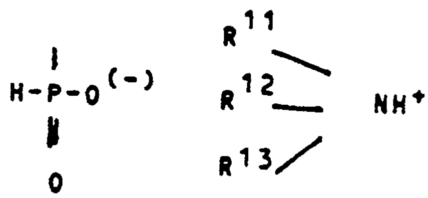

worin $R^{11}$, $R^{12}$ und $R^{13}$, die gleich oder verschieden sein können, Alkylreste darstellen; und $R^5$ ein Wasserstoffatom,

dadurch gekennzeichnet, daß es die folgenden aufeinanderfolgenden Stufen umfaßt:

a) Reaktion eines Nucleosids, ausgewählt aus den Nucleosiden von Guanin, Cytosin und Adenin, mit einem Säurechlorid der Formel

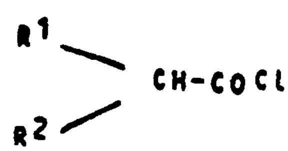

oder einem Säureanhydrid der Formel

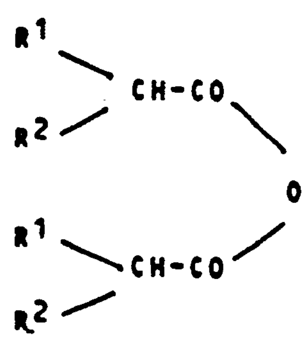

worin $R^1$ und $R^2$ wie oben definiert sind, unter Bildung eines Nucleosidderivats der Formel (I), worin $R^3$ und $R^4$ den Rest der Formel darstellen:

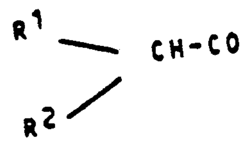

worin $R^1$ und $R^2$ wie oben definiert sind, und $R^5$ ein Wasserstoffatom bedeutet;

b) Eliminierung der Reste $R^3$ und $R^4$ aus dem in der Stufe (a) erhaltenen Nucleosidderivat durch selektive Hydrolyse unter Bildung des Nucleosidderivats der Formel (I), worin $R^3$ und $R^4$ ein Wasserstoffatom darstellen;

c) Reaktion des in der Stufe (b) erhaltenen Nucleosidderivats mit einem Tritylchlorid der Formel:

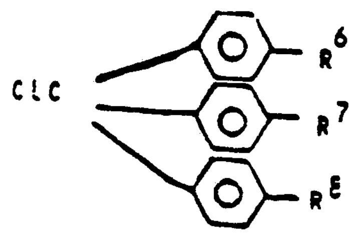

worin $R^6$, $R^7$ und $R^8$` die gleich oder verschieden sein können, ein Wasserstoffatom, einen Alkylrest oder einen Alkoxyrest bedeuten,
unter Bildung des Nucleosidderivats der Formel (I), worin $R^3$ einen Tritylrest darstellt;

d) Reaktion des in der Stufe (c) erhaltenen Nucleosidderivats mit dem 4-Chlor-phenylphosphoryl-bistriazolidat oder dem $\beta$-Cyanoethyl-monochloro-N,N-diisopropylaminphosphoramidit oder dem 2-Chloro-(5,6a)benzo-1,3-dioxo-2-phosphor-4-inon und einem Trialkylammoniumsalz unter Bildung des Nucleosidderivats der Formel (I), worin $R^3$ einen Tritylrest, $R^4$ einen Rest, ausgewählt aus den Resten der Formel:

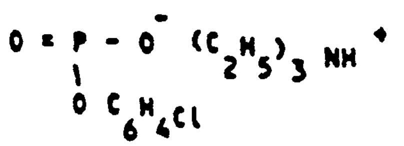 (VI)

der Formel:

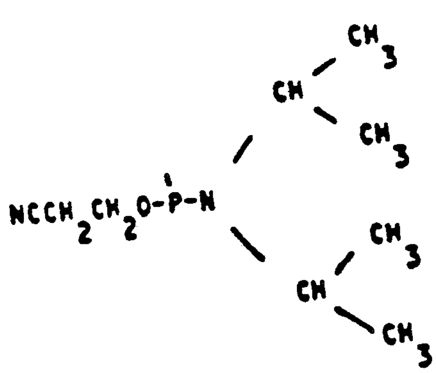 (VII)

oder der Formel:

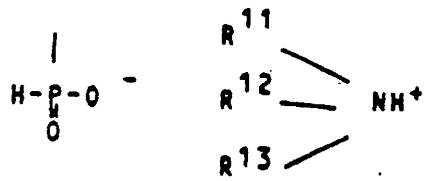

und $R^5$ ein Wasserstoffatom darstellen.

3. Verfahren zur Synthese von Oligonucleotiden, dadurch gekennzeichnet, daß es umfaßt:

1) mindestens einen Kondensationscyclus, in dem man ein Nucleosidderivat der Formel

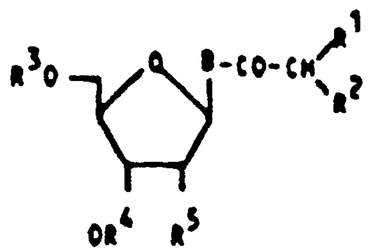

(I)

worin bedeuten:

B einen bivalenten Rest, ausgewählt aus der Gruppe:

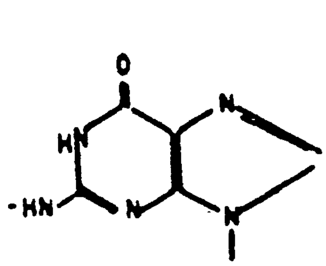

(II) ;

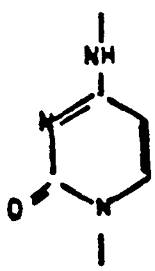

(III) oder

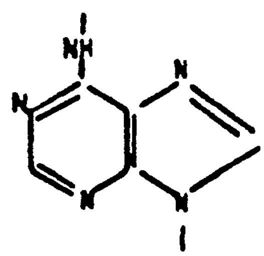

(IV)

der über seine exocyclische NH-Gruppe an die CO-Gruppe gebunden ist;

$R^1$ ein Wasserstoffatom oder einen Methyl- oder Ethylrest;

$R^2$ einen Rest, ausgewählt aus der Gruppe, die besteht aus den Resten Methoxy und Phenoxy, unsubstituiert oder substituiert durch eine oder mehrere der Gruppen, ausgewählt aus $NO_2$, CN, Methoxy, Ethoxy, Phenoxy, Cl, F,

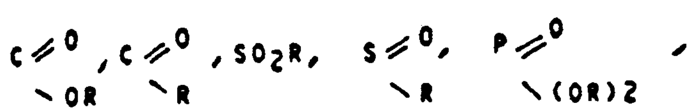

Methyl, Ethyl oder Phenyl, substituiert oder unsubstituiert, SR, worin R für einen Methyl-, Ethyl- oder Phenylrest steht;

$R^3$ einen in saurem Milieu labilen Rest;

$R^4$ einen phosphorhaltigen Rest; und

$R^5$ ein Wasserstoffatom;

an ein Nucleosidderivat oder an ein Oligonucleotid ankondensiert und

2) eine Stufe zur Eliminierung der Schutzgruppe(n) der Formel:

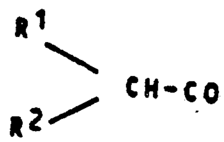

worin $R^1$ und $R^2$ wie oben definiert sind.

4. Verfahren nach Anspruch 3, dadurch gekennzeichnet, daß der in saurem Milieu labile Rest $R^3$ ausgewählt wird aus der Gruppe der Tritylreste der Formel

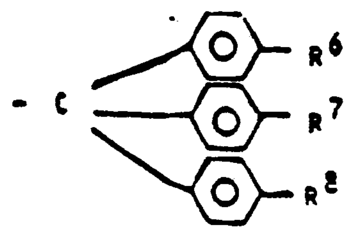

(V)

worin $R^6$, $R^7$ und $R^8$, die gleich oder verschiedcen sein können, ein Wasserstoffatom, einen Alkylrest oder einen Alkoxyrest darstellen, der Pixylreste und der 9-Phenyl-xanthenylreste.

5. Verfahren nach einem der Ansprüche 3 und 4, dadurch gekennzeichnet, daß der phosphorhaltige Rest $R^4$ ausgewählt wird aus der Gruppe der Reste der Formel:

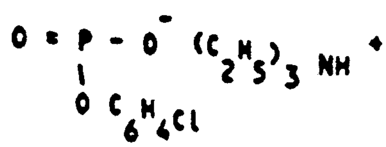

(VI)

der Formel:

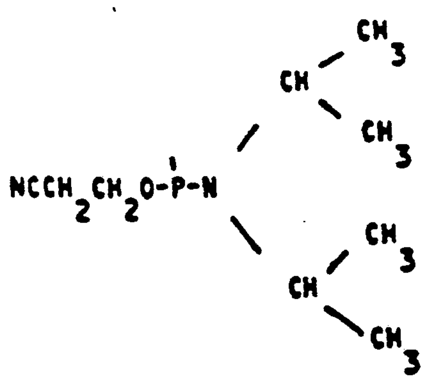

(VII)

oder der Formel:

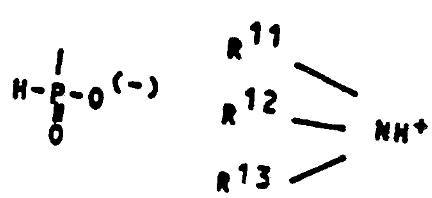

worin $R^{11}$, $R^{12}$ und $R^3$, die gleich oder verschieden sein können, Alkylreste darstellen.

6. Verfahren nach einem der Ansprüche 3 bis 5, dadurch gekennzeichnet, daß B darstellt:

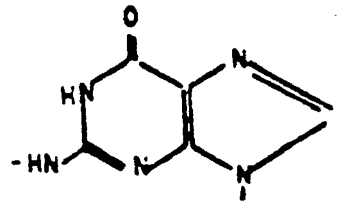

(II)

$R^1$ ein Wasserstoffatom darstellt und

$R^2$ einen Rest darstellt, ausgewählt aus der Gruppe:

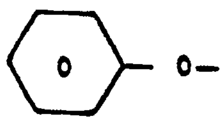 , 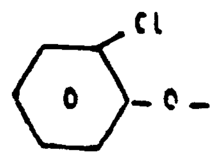 oder $CH_3O-$

7. Verfahren nach einem der Ansprüche 3 bis 5, dadurch gekennzeichnet, daß B darstellt

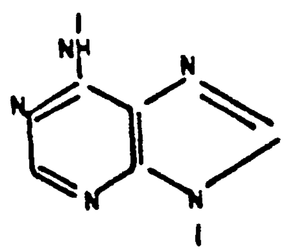

(IV)

$R^1$ ein Wasserstoffatom darstellt und

$R^2$ den Phenoxyrest darstellt.

8. Verfahren nach einem der Ansprüche 6 und 7, dadurch gekennzeichnet, daß $R^3$ den Rest darstellt:

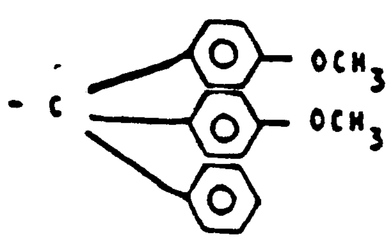

und $R^4$ den Rest darstellt:

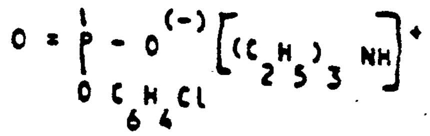 (VI)

9. Verfahren nach einem der Ansprüche 6 und 7, dadurch gekennzeichnet, daß $R^3$ den Rest darstellt:

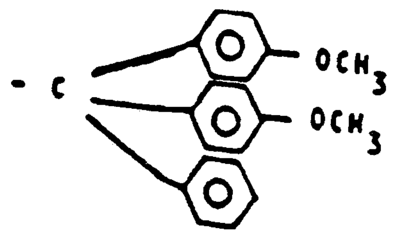

und $R^4$ den Rest darstellt:

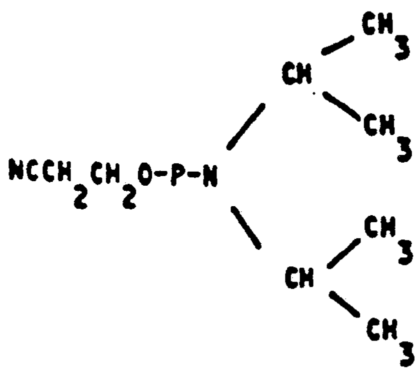 (VII)

10. Verfahren nach einem der Ansprüche 6 und 7, dadurch gekennzeichnet, daß $R^3$ den Rest der Formel darstellt:

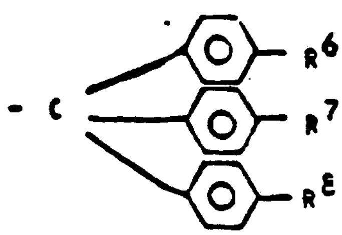 (V)

und $R^4$ den Rest darstellt: